# EUROPÄISCHE PATENTSCHRIFT

(11) **EP 1 207 866 B1**
(45) Veröffentlichungstag und Bekanntmachung des Hinweises auf die Patenterteilung: **13.10.2004**
(21) Anmeldenummer: 00949447.7
(22) Anmeldetag: 03.08.2000
(51) Int. Cl.: A61K 31/135, A61K 9/32, A61K 9/52

(54) **RETARDIERTE DARREICHUNGSFORM ENTHALTEND TRAMADOLSACCHARINAT**
DELAYED-ACTION FORM OF ADMINISTRATION CONTAINING TRAMADOL SACCHARINATE
FORME D'ADMINISTRATION A ACTION RETARDEE CONTENANT DU SACCHARINATE DE TRAMADOL

(30) Priorität: 31.08.1999 DE 19940944; 31.08.1999 DE 19940740; 16.05.2000 DE 10023699
(43) Veröffentlichungstag der Anmeldung: 29.05.2002
(73) Patentinhaber: Grünenthal GmbH, 52078 Aachen (DE)
(72) Erfinder: BARTHOLOMÄUS, Johannes, D-52080 Aachen (DE); KUGELMANN, Heinrich, D-52080 Aachen (DE); ZIEGLER, Iris, D-52159 Rott-Roetgen (DE)
(86) Internationale Anmeldenummer: PCT/EP2000/007527
(87) Internationale Veröffentlichungsnummer: WO 2001/015683

(56) Entgegenhaltungen:
- DE-A- 19 530 575
- DE-A- 19 630 035

## Beschreibung

Die vorliegende Erfindung betrifft durch einen Überzug retardierte Tramadol-Darreichungsformen, die den Wirkstoff Tramadol als Tramadolsaccharinat sowie ggf. weitere Hilfsstoffe enthalten.

Das sehr gut wasserlösliche Tramadolhydrochlorid - (1RS,2RS)-2-[(Dimethylamino)methyl]-1-(3-methoxyphenyl)-cyclohexanol-Hydrochlorid - wird häufig zur Bekämpfung starker und mittelstarker Schmerzen verwendet.

Die Applikation des Tramadolhydrochlorids in Form retardierter Zubereitungen stellt für diesen Wirkstoff eine Therapieverbesserung dar. Die Retardierung ermöglicht es, auch für diesen Wirkstoff mit einer relativ kurzen Halbwertszeit im Organismus, eine Zubereitung mit lang anhaltender Wirkung zur Verfügung zu stellen und durch gleichmäßigere Blutspiegel außerdem Nebenwirkungen zu vermindern sowie die Einhaltung der Dosierungsvorschrift bei den Patienten zu verbessern.

Die Retardierung des Wirkstoffes Tramadolhydrochlorid kann z.B. durch das Überziehen von Tramadolhydrochlorid-haltigen Arzneiformen mit retardierenden Filmüberzügen erreicht werden. Eine Retardierung dieses Wirkstoffes mit Hilfe von Filmüberzügen ist jedoch mit einem relativ hohen Aufwand verbunden, da Filmüberzüge aus wäßrigen Überzugssystemen für solche sehr gut wasserlöslichen Wirkstoffe häufig nur eine unzureichende Diffusionsbarriere darstellen und sich die Permeabilität dieser Filmüberzüge für Tramadolhydrochlorid während der Lagerung meistens ändert (P.B. O'Donnell, J.W. McGinity, "Mechanical Properties of Polymeric Films, Prepared from Aqueous Polymeric Dispersions in Aqueous Polymeric Coatings for Pharmaceutical Dosage Forms", Drugs and the Pharmaceutical Science Vol. 79, ed. J.W. McGinity, Marcel Decker, New York, Basel, Hong Kong 1997).

Die Herstellung dieser retardierten Tramadolhydrochlorid-Zubereitungen erfordert daher relativ aufwendige Überzugsverfahren mit mehrschichtigen Filmen oder zeitintensive Temperverfahren, wie sie in der US-PS-5,645,858 bzw. in den US-PS 5,580,578, US-PS 5,681,585, US-PS-5,472,712 sowie in K. Bauer, "Coated Pharmaceutical Dosage Forms", medpharm Scientific Publishers, Stuttgart 1998, B. Sutter, Dissertation, Universität Düsseldorf, 1987 oder in F.N. Christensen, Proceed. Intern. Symp. Contr. Rel. Bioact. Mater. 17, 124, 1990 beschrieben sind. Sofern solche Überzüge aus organischen Lösungsmitteln aufgebracht werden, verteuert die damit verbundene Umwelt- und Lösungsmittelrückstandproblematik zusätzlich die Retardierung von Tramadolhydrochlorid.

Die der Erfindung zugrundeliegende Aufgabe bestand daher darin, eine mit Hilfe eines Überzugs retardierte Darreichungsform des Wirkstoffes Tramadol zur Verfügung zu stellen, die unmittelbar nach ihrer Herstellung ein lagerstabiles Wirkstoff-Freisetzungsprofil aufweist, ohne daß aufwendige und kostspielige Überzugsverfahren oder zeit- und daher kostenintensive Temperverfahren erforderlich sind.

Erfindungsgemäß wird diese Aufgabe durch die Bereitstellung von mit einem retardierenden Überzug versehenen Darreichungsformen gelöst, die den Wirkstoff Tramadol als Tramadolsaccharinat sowie ggf. weitere Hilfsstoffe enthalten.

Die erfindungsgemäßen, retardierten Darreichungsformen zeigen überraschenderweise bereits unmittelbar nach ihrer Herstellung ein lagerstabiles Wirkstoff-Freisetzungsprofil, ohne daß eine sich an die übliche Trocknung anschließende Temperung des retardierenden Überzuges erforderlich ist.

Zur Herstellung des Tramadolsaccharinates wird als Tramadol (1RS,2RS)-2-[(Dimethylamino)methyl]-1-(3-methoxyphenyl)-cyclohexanol und/oder wenigstens ein entsprechendes, vorzugsweise wasserlösliches Salz mit Saccharin und/oder wenigstens einem, vorzugsweise wasserlöslichem Salz des Saccharins umgesetzt. Als Salz des Tramadols wird bevorzugt Tramadolhydrochlorid, als Salz des Saccharins bevorzugt dessen Natrium-, Kalium-, Kalzium- oder Ammoniumsalz, besonders bevorzugt dessen Natriumsalz eingesetzt.

Das Tramadolsaccharinat kann auch in situ bei der Herstellung der Darreichungsformen gebildet werden.

In situ Bildung im Sinne der vorliegenden Erfindung bedeutet, daß ein leicht wasserlösliches Salz des Tramadols mit einem wasserlöslichen Salz des Saccharins gemischt, mehrfach angefeuchtet und granuliert, gegebenenfalls extrudiert und/oder unter sonstigem Energieeintrag, vorzugsweise unter Druck und/oder Wärmezufuhr formuliert wird.

Zur in situ Bildung des Tramadolsaccharinates kann das Tramadol als wasserlösliches, pharmazeutisch verträgliches Salz, bevorzugt als Tramadolhydrochlorid und als wasserlösliches, pharmazeutisch verträgliches Salz des Saccharins, bevorzugt dessen Natrium-, Kalium-, Kalzium- oder Ammoniumsalz, besonders bevorzugt dessen Natriumsalz eingesetzt werden.

Die erfindungsgemäßen, mit einem retardierenden Überzugsfilm versehenen Darreichungsformen eignen sich bevorzugt zur oralen Applikation.

In einer bevorzugten Ausführungsform der vorliegenden Erfindung liegen die erfindungsgemäßen Darreichungsformen in Form von Tabletten, Kapseln oder Suspensionen vor.

In einer weiteren bevorzugten Ausführungsform der vorliegenden Erfindung liegen die erfindungsgemäßen Darreichungsformen in multipartikulärer Form, vorzugsweise in Form von Mikrotabletten, Mikrokapseln, Mikropellets, Granulaten, Wirkstoffkristallen oder Pellets, ggf. in Kapseln abgefüllt oder zu Tabletten verpreßt, oder in einer hydrophilen oder lipophilen Flüssigkeit, vorzugsweise homogen suspendiert, besonders bevorzugt in Form von Säften oder Oraldispersionen, vor. Sofern die erfindungsgemäßen Darreichungsformen in Form von Granulaten oder Pellets vorliegen, können diese vorzugsweise eine Größe im Bereich von 0,1 bis 3 mm, besonders bevorzugt im Bereich von 0,5 bis 2 mm aufweisen.

Liegen die erfindungsgemäßen Darreichungsformen in Form von Mikrotabletten vor, können diese bevorzugt einen Durchmesser im Bereich von 0,5 bis 5 mm, besonders bevorzugt im Bereich von 1 bis 3 mm und ganz besonders bevorzugt im Bereich von 1 bis 2 mm aufweisen.

Liegen die erfindungsgemäßen Darreichungsformen in Form von Wirkstoffkristallen, Mikropartikeln, Mikropellets oder Mikrokapseln vor, so können diese bevorzugt einen Durchmesser im Bereich von 10 µm bis 1 mm, besonders bevorzugt im Bereich von 15 µm bis 0,5 mm und ganz besonders bevorzugt im Bereich von 30 µm bis 200 µm aufweisen.

Die erfindungsgemäßen Darreichungsformen können außerdem je nach Ausführungsform als weitere Bestandteile die üblichen, dem Fachmann bekannten Hilfsstoffe enthalten.

Sofern die erfindungsgemäßen Darreichungsformen in Form von Tabletten oder Mikrotabletten vorliegen, können diese als weitere Hilfsstoffe vorzugsweise mikrokristalline Cellulose, Celluloseether, Lactose, Stärke und Stärkederivate, Zuckeralkohole, Calciumhydrogenphosphat sowie die üblichen, dem Fachmann bekannten Bindemittel, Fließregulationsmittel, Gleitmittel und gegebenenfalls Sprengmittel enthalten.

Liegen die erfindungsgemäßen Darreichungsformen in Form von Pellets, Granulaten oder Mikropellets vor, können diese als weitere Hilfsstoffe bevorzugt mikrokristalline Cellulose, Celluloseether, Lactose, Stärke und Stärkederivate, Zuckeralkohole, Calciumhydrogenphosphat, Fettalkohole, Ester des Glycerins oder Fettsäureester enthalten.

Liegen die erfindungsgemäßen Darreichungsformen in Form von Mikrokapseln oder Mikropartikeln vor, so können diese je nach Art des zu ihrer Herstellung eingesetzten Verfahrens die üblichen, dem Fachmann bekannten Hilfsstoffe enthalten.

Die Herstellung der verschiedenen erfindungsgemäßen Darreichungsformen kann nach verschiedenen, dem Fachmann bekannten Methoden erfolgen.

Sofern die erfindungsgemäße Darreichungsform in Form von Tabletten vorliegt, können diese beispielsweise durch das Verpressen mittels Feucht-, Trocken-. oder Schmelzgranulation hergestellter Granulate oder durch direkte Tablettierung des Tramadolsaccharinates, ggf. mit weitereren Hilfsstoffen hergestellt werden. Desweiteren können die Tabletten durch Verpressen überzogener Pellets, Wirkstoffkristalle, Mikropartikel oder Mikrokapseln hergestellt werden.

Die erfindungsgemäße Darreichungsform in Form von Pellets kann durch Extrusion und Spheronisation, durch Aufbaupelletisierung oder durch Direktpelletisierung in einem hochtourigen Mischer oder in der Rotorwirbelschicht hergestellt werden. Besonders bevorzugt ist die Herstellung der Pellets durch Extrusion feuchter Massen und anschließender Spheronisation.
Die Herstellung von Mikrokapseln erfolgt nach üblichen Mikroverkapselungsverfahren, wie z.B. durch Sprühtrocknung, Sprüherstarrung oder Koazervation.

In einer bevorzugten Ausführungsform der erfindungsgemäßen Darreichungsformen basiert der retardierende Überzugsfilm vorzugsweise auf einem wasserunlöslichen, gegebenenfalls modifizierten natürlichen und/oder synthetischen Polymeren oder auf einem natürlichen, halbsynthetischen oder synthetischen Wachs oder auf einem Fett oder einem Fettalkohol oder auf einem Gemisch aus wenigstens zwei der vorstehend genannten Komponenten.

Als wasserunlösliche Polymere werden zur Herstellung eines retardierenden Überzugs vorzugsweise Poly(meth)acrylate, besonders bevorzugt Poly(C₁₋₄)-Alkyl(meth)acrylate, Poly(C₁₋₄)-dialkylamino-(C₁₋₄)-alkyl(meth)acrylate und/oder deren Copolymere, ganz besonders bevorzugt Copolymere aus Ethylacrylat und Methylmethacrylat mit einem molaren Verhältnis der Monomeren von 2 : 1 (Eudragit NE30D®), Copolymere aus Ethylacrylat, Methylmethacrylat und Trimethylammoniumethylmethacrylat-chlorid mit einem molaren Verhältnis der Monomeren von 1 : 2 : 0,1 (Eudragit RS®), Copolymere aus Ethylacrylat, Methylmethacrylat und Trimethylammoniumethylmethacrylatchlorid mit einem molaren Verhältnis der Monomeren von 1 : 2 : 0,2 (Eudragit RL®) oder ein Gemisch aus wenigstens zwei dieser vorstehend genannten Copolymeren eingesetzt. Diese Überzugsmaterialien sind als 30 Gew.%-ige wäßrige Latexdispersionen, d.h. als Eudragit RS30D®, Eudragit NE30D® bzw. Eudragit RL30D® am Markt erhältlich und werden als solche auch als Überzugsmaterial bevorzugt eingesetzt.

Ebenfalls bevorzugt können als wasserunlösliche Polymere zur Herstellung des retardierenden Überzugs der erfindungsgemäßen Darreichungsformen Polyvinylacetate ggf. in Kombination mit weiteren Hilfstoffen eingesetzt werden. Diese sind als wäßrige Dispersion enthaltend 27 Gew.-% Polyvinylacetat, 2.5 Gew.-% Povidon und 0,3 Gew.-% Natriumlaurylsulfat (Kollicoat SR 30 D®) am Markt erhältlich.

In einer weiteren bevorzugten Ausführungsform basieren die retardierenden Überzüge der erfindungsgemäßen Darreichungsformen auf wasserunlöslichen Cellulosederivaten, vorzugsweise Alkylcellulosen wie z.B. Ethylcellulose, oder Celluloseestern, wie z.B. Celluloseacetat. Die Überzüge aus Ethylcellulose oder Celluloseacetat werden bevorzugt aus wäßriger Pseudolatexdispersion aufgebracht. Wäßrige Ethylcellulose-Pseudolatexdispersionen werden als 30 Gew.%-ige Dispersionen (Aquacoat®) oder als 25 Gew.%-ige Dispersionen (Surelease®) am Markt geführt.

Als natürliche, halbsynthetische oder synthetische Wachse, Fette bzw. Fettalkohole kann der retardierende Überzug der erfindungsgemäßen Darreichungsformen, vorzugsweise auf Carnaubawachs, Bienenwachs, Glycerinmonostearat, Glycerinmonobehenat (Compritol ATO888®), Glycerinditripalmitostearat (Precirol ATO5®), mikrokristallines Wachs, Cetylalkohol, Cetylstearylalkohol oder einem Gemisch aus wenigstens zwei dieser Komponenten basieren.

Sofern der retardierende Überzug auf einem wasserunlöslichen, gegebenenfalls modifizierten natürlichen und/oder synthetischen Polymeren basiert, kann die Überzugsdispersion oder Lösung neben dem entsprechendem Polymer einen üblichen, dem Fachmann bekannten, physiologisch verträglichen Weichmacher aufweisen, um die notwendige Mindestfilmtemperatur zu senken.

Geeignete Weichmacher sind beispielsweise lipophile Diester aus einer aliphatischen oder aromatischen Dicarbonsäure mit C₆-C₄₀ und einem aliphatischen Alkohol mit C₁-C₈, wie z.B. Dibutylphthalat, Diethylphthalat, Dibutylsebacat oder Diethylsebacat, hydrophile oder lipophile Ester der Zitronensäure, wie z.B. Triethylcitrat, Tributylcitrat, Acetyltributylcitrat oder Acetyltriethylcitrat, Polyethylenglycole, Propylenglycol, Ester des Glycerins, wie z.B. Triacetin, Myvacet® (acetylierte Mono- und Diglyceride, C₂₃H₄₄O₅ bis C₂₅H₄₇O₇), mittelkettige Triglyceride (Miglyol®), Ölsäure oder Gemische aus wenigstens zwei der genannten Weichmacher.
Vorzugsweise enthalten wäßrige Dispersionen von Eudragit RS® und gegebenenfalls Eudragit RL® Triethylcitrat.

Vorzugsweise enthält der retardierende Überzug der erfindungsgemäßen Darreichungsform den/die Weichmacher in Mengen von 5 bis 50 Gew-%., besonders bevorzugt 10 bis 40 Gew.-% und ganz besonders bevorzugt 10 bis 30 Gew-%, bezogen auf die Menge an des/der eingesetzten Polymer(en). In Einzelfällen, beispielsweise für Celluloseacetat können auch höhere Mengen an Weichmachern, vorzugsweise bis zu 110 Gew.-% eingesetzt werden.

Desweiteren kann der retardierende Überzug weitere übliche, dem Fachmann bekannte Hilfsstoffe, wie z.B. Gleitmittel, vorzugsweise Talkum oder Glycerinmonostearat, Farbpigmente, vorzugsweise Eisenoxide oder Titandioxid, oder Tenside, wie z.B. Tween 80® aufweisen.

Das umittelbar nach der Herstellung der erfindungsgemäßen Darreichungsform erhaltene Freisetzungsprofil von Tramadol kann durch die üblichen, dem Fachmann bekannten Methoden, wie z.B. durch die Dicke des Überzugs oder durch den Einsatz weiterer Hilfsstoffe als Bestandteile des Überzugs eingestellt werden. Geeignete Hilfsstoffe sind beispielsweise hydrophile oder pH-abhängige Porenbildner, wie z.B.

Natrium-Carboxymethylcellulose, Celluloseacetatphthalat, Hydroxypropylmethylcelluloseacetatsuccinat, Lactose, Polyethylenglykol oder Mannitol oder wasserlösliche Polymere, wie z.B. Polyvinylpyrrolidon oder wasserlösliche Cellulosen, vorzugsweise Hydroxypropylmethylcellulose oder Hydroxypropylcellulose.

Der retardierende Überzug kann auch unlösliche bzw. lipophile Hilfsstoffe, wie z.B. alkylisiertes Siliciumdioxid, das z.B. als Aerosil R972® am Markt geführt wird, oder Magnesiumstearat zur weiteren Verstärkung der Retardierung enthalten.

Die erfindungsgemäßen Darreichungsformen zur Freisetzung des Tramadolsaccharinates können zusätzlich auch einen magensaftresistenten Überzug aufweisen, der sich pH-abhängig auflöst. Durch diesen Überzug kann erreicht werden, daß die erfindungsgemäßen Darreichungsformen den Magentrakt unaufgelö'st passieren und das Tramadolsaccharinat erst im Darmtrakt zur Freisetzung gelangt. Vorzugsweise löst sich der magensaftresistente Überzug bei einem pH-Wert zwischen 5 und 7,5 auf.
Der magensaftresistente Überzug basiert vorzugsweise auf Methacrylsäure/Methylmethacrylat-Copolymeren mit einem molaren Verhältnis der jeweiligen Monomeren von 1 : 1 (Eudragit L®), Methacrylsäure/Methylmethacrylat-Copolymeren mit einem molaren Verhältnis der jeweiligen Monomeren von 1 : 2 (Eudragit S®), Methacrylsäure/Ethylacrylat-Copolymeren mit einem molaren Verhältnis der jeweiligen Monomeren von 1: 1 (Eudragit L30D-55®) Methacrylsäure/Methylacrylat/Methylmethacrylat-Copolymeren mit einem molaren Verhältnis der jeweiligen Monomeren von 7 : 3 : 1 (Eudragit FS®), Schellack Hydroxypropylmethylcelluloseacetatsuccinate, Celluloseacetatphthalate oder einer Mischung aus wenigstens zwei dieser Komponenten, die ggf. auch in Kombination mit den vorstehend genannten wasserunlöslichen Poly(meth)acrylaten, vorzugsweise in Kombination mit Eudragit NE30D® und/oder Eudragit RL® und/oder Eudragit RS® eingesetzt werden können.

Die Überzüge der erfindungsgemäßen Darreichungsform können nach den üblichen, für den jeweiligen Überzug geeigneten, dem Fachmann bekannten Verfahren, wie z.B. durch Aufsprühen von Lösungen, Dispersionen oder Suspensionen, durch Schmelzverfahren oder durch Pulverauftragsverfahren aufgebracht werden. Die Lösungen, Dispersionen oder Suspensionen können in Form von wäßrigen oder organischen Lösungen oder Dispersionen eingesetzt werden. Dabei werden wäßrige Dispersionen bevorzugt eingesetzt. Als organische Lösungsmittel können Alkohole, beispielsweise Ethanol oder Isopropanol, Ketone, wie z.B. Aceton, Ester, beispielsweise Ethylacetat, chlorierte Kohlenwasserstoffe, wie z.B. Dichlormethan verwendet werden, wobei Alkohole und Ketone bevorzugt eingesetzt werden. Es ist auch möglich Mischungen aus wenigstens zwei der vorstehend genannten Lösungsmittel einzusetzen.
Diese Verfahren sind aus dem Stande der Technik, z.B. H. Sucker, Georg Thieme Verlag, 1991, Seiten 347 ff. bekannt.

Sofern die erfindungsgemäßen Darreichungsformen in multipartikulärer Form vorliegen, wird der retardierende Überzug vorzugsweise so aufgebracht, daß man die multipartikulären Formen enthaltend das Tramadolsaccharinat nach ihrer Herstellung mit den jeweiligen Polymeren und ggf. weiteren Hilfsstoffen aus wässrigen und/oder organischen Medien, vorzugsweise aus wässrigen Medien, mit Hilfe des Wirbelschichtverfahrens überzieht und den Überzug vorzugsweise gleichzeitig bei üblichen Temperaturen in der Wirbelschicht trocknet, ohne den Überzug anschließend zu tempern. Vorzugsweise erfolgt die Trocknung des Überzuges für Poly(meth)acrylatüberzüge bei einer Zulufttemperatur im Bereich von 30 bis 50 °C, besonders bevorzugt im Bereich von 35 bis 45 °C.

Für Überzüge auf Cellulosebasis, wie z.B. Ethylcellulose oder Celluloseacetat, erfolgt die Trocknung bevorzugt bei einer Temperatur im Bereich von 50 bis 80 °C, besonders bevorzugt im Bereich von 55 bis 65 °C.

Wachsüberzüge können durch Schmelzüberziehen in der Wirbelschicht aufgebracht werden und bei Temperaturen unterhalb des jeweiligen Schmelzbereiches nach dem Überziehen zur vollständigen Verfestigung abgekühlt werden. Das Aufbringen von Wachsüberzügen kann auch durch Aufsprühen von deren Lösungen in organischen Lösungsmitteln erfolgen.

Zur weiteren Modifizierung des Wirkstoff-Freisetzungsprofils können die erfindungsgemäßen, retardierten Darreichungsformen Tramadolsaccharinat auch in einer retardierenden Matrix, vorzugsweise gleichmäßig verteilt, enthalten.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Diglyceride von C₁₂-C₃₀-Fettsäuren und/oder C₁₂-C₃₀-Fettalkohole und/oder Wachse oder deren Gemische eingesetzt.

Es ist auch möglich, Mischungen der vorstehend genannten hydrophilen und hydrophoben Materialien als retardierendes Matrixmaterial einzusetzen.

Die Herstellung der retardierenden Matrix kann nach den üblichen, dem Fachmann bekannten Methoden erfolgen.

In einer weiteren bevorzugten Ausführungsform enthalten die erfindungsgemäßen Darreichungsformen den Wirkstoff Tramadol neben seiner retardierten Form auch in seiner unretardierten Form, z.B. als Tramadolhydrochlorid. Durch Kombination mit dem sofort freigesetzten Wirkstoff läßt sich eine hohe Initialdosis zur schnellen Schmerzlinderung erzielen. Die langsame Freisetzung aus der retardierten Form verhindert dann ein Abklingen der analgetischen Wirkung.

Die an den Patienten zu verabreichende Menge des Tramadols variiert z.B. in Abhängigkeit vom Gewicht des Patienten, von der Indikation sowie dem Schweregrad der Schmerzen bzw. der Erkrankung. Vorzugsweise wird die zu verabreichende Menge des Tramadols sowie dessen Freisetzung so eingestellt, daß eine Applikation höchstens zweimal, vorzugsweise nur einmal täglich erfolgen muß.

Bei einer einmaligen Applikation pro Tag enthalten die erfindungsgemäßen Darreichungsformen bevorzugt 10 bis 1200 mg, besonders bevorzugt 20 bis 1000 mg und ganz besonders bevorzugt 100 bis 800 mg Tramadol.

Bei einer zweimaligen Applikation pro Tag enthalten die erfindungsgemäßen Darreichungsformen vorzugsweise 5 bis 600 mg, besonders bevorzugt 10 bis 500 mg und ganz besonders bevorzugt 50 bis 400 mg Tramadol.

Vorzugsweise können die erfindungsgemäßen Darreichungsformen zur Bekämpfung von Schmerzen oder zur Behandlung von Harninkontinenz, Husten, inflammatorischen Reaktionen, allergischen Reaktionen, Depressionen, Drogenmißbrauch, Alkoholmißbrauch, Gastritis, Diarrhoe, cardiosvaskulären Erkrankungen, Atemwegserkrankungen, seelischen Erkrankungen oder Epilepsie, besonders bevorzugt zur Bekämpfung von Schmerzen oder zur Behandlung von Harninkontinenz oder Husten eingesetzt werden.

Die erfindungsgemäßen Darreichungsformen haben den Vorteil, daß sie bereits unmittelbar nach ihrer Herstellung ein lagerstabiles Wirkstoff-Freisetzungsprofil aufweisen, ohne daß eine nach der Trocknung üblicherweise anschließende Temperung oder ein aufwendiges Überzugsverfahren erforderlich ist. Dies ermöglicht es, die Produktionsdauer und damit auch die Kosten für die Herstellung der erfindungsgemäßen Darreichungsformen zu verringern.

Darüber hinaus erfolgt die Freisetzung des Wirkstoffes Tramadol überraschenderweise aus den erfindungsgemäßen, mit einem aus wäßrigem Medium aufgetragenen, retardierenden Überzug versehenen Darreichungsformen weit stärker retardiert als aus den mit einem identisch aufgebauten retardierenden Überzug versehenen Darreichungsformen, die den Wirkstoff Tramadol als Tramadolhydrochlorid enthalten. Das Aufbringen des retardierenden Überzugfilms aus wäßrigem Medium hat weiterhin den Vorteil, daß eine aufwendige Rückgewinnung organischer Lösemittel nicht erforderlich ist, und daß die erfindungsgemäßen Darreichungsformen keine Lösemittelrückstände mehr enthalten.

Desweiteren zeichnen sich die erfindungsgemäßen Darreichungsformen dadurch aus, daß die Freisetzung des Wirkstoffes Tramadol aus den erfindungsgemäßen Darreichungsformen durch eine Variation der Freisetzungsbedingungen im üblichen Rahmen, wie z.B. durch die lonenkonzentrationen der Puffer, durch die Anwesenheit von oberflächenaktiven Substanzen oder durch unterschiedliche mechanische Belastung nicht beeinflußt wird.

Die Freisetzungsprofile der erfindungsgemäßen Darreichungsformen wurde wie folgt bestimmt:

Die erfindungsgemäße Darreichungsform wurde in der Körbchenapparatur oder der Blattrührerapparatur des Pharm. Eur. bei einer Temperatur des Freisetzungsmediums von 37 ± 0,5 °C bei einer Umdrehungsgeschwindigkeit von 100 Umdrehungen pro Minute bzw. 50 Umdrehungen pro Minute im Falle der Blattrührapparatur 2 Stunden lang in 600 ml künstlichem Magensaft bei pH 1,2 geprüft. Anschließend wurde die Darreichungsform weitere 8 Stunden lang in 900 ml künstlichem Darmsaft bei pH 7,2 geprüft. Die jeweils zu einem Zeitpunkt freigesetzte Menge an Tramadolsaccharinat wurde mittels HPLC bestimmt. Die dargestellten Werte sind die Mittelwerte aus jeweils 3 Proben.

im folgenden wird die Erfindung anhand der Beispiele 1 bis 11 erläutert. Diese Erläuterungen sind lediglich beispielhaft und schränken den allgemeinen Erfindungsgedanken nicht ein.

### Beispiele:

### Beispiel 1:

Zur Herstellung des Tramadolsaccharinates werden äquimolare Mengen von Tramadol (1RS,2RS)-2-[(Dimethylamino)methyl]-1-(3-methoxyphenyl)-cyclohexanolhydrochlorid und Saccharin-Natrium oder Saccharin-Natrium-Dihydrat jeweils unter Erwärmen in einer möglichst geringen Menge Wasser vollständig gelöst. Anschließend werden beide Lösungen unter Rühren miteinander vermischt. Das Tramadolsaccharinat kristallisiert beim Abkühlen aus der wäßrigen Lösung schon nach kurzer Zeit aus und wird nach herkömmlichen Methoden isoliert und mit Ethanol gereinigt.

### Herstellung der Pellets:

2500 g Tramadolsaccharinat und 2500 g mikrokristalline Cellulose (Avicel PH 105®, FMC) werden für 10 Minuten in einem Diosna P25 Schnellmischer gemischt und anschließend mit 3300 g demineralisiertem Wasser für 10 Minuten granuliert. Das feuchte Granulat wird im NICA E140 Extruder mit einer 1,0 x 2,0 mm Extrusionsmatrize extrudiert und das feuchte Extrudat in Chargen von 2,7 kg im NICA Spheronizer 15 Minuten bei 800 Umdrehungen pro Minute (UPM) spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

3 kg dieser Pellets (800 -1250 µm) werden in der Wirbelschicht (Hüttlin HKC05) mit einer wäßrigen Dispersion der nachstehenden Zusammensetzung bei einer Produkttemperatur von 26 bis 30°C bis zu einer Gewichtszunahme von 16 Gew.-% (bezogen auf das Gewicht der Ausgangspellets) überzogen. Dieser Überzugsauftrag entspricht einem Polymerauftrag von 13 Gew.-% (bezogen auf das Gewicht der Ausgangspellets). Die Pellets werden anschließend 10 Minuten in der Wirbelschicht bei 45°C getrocknet.

| Wäßrige Dispersion für 3 kg Pellets: | |
|---|---|
| Eudragit RS 30 D® | 1049,0 g |
| Eudragit RL 30 D® | 296,0 g |
| Triethylcitrat | 80,7 g |
| Glycerinmonostearat | 20,2 g |
| Tween 80® | 1,9 g |
| Demineralisiertes Wasser | 1074,2 g |
| Gesamt: | 2522,0 g |

345 mg mit Retardüberzug versehene Pellets enthalten 149 mg Tramadolsaccharinat, entsprechend einer Wirkstoffmenge von 100 mg Tramadolhydrochlorid.

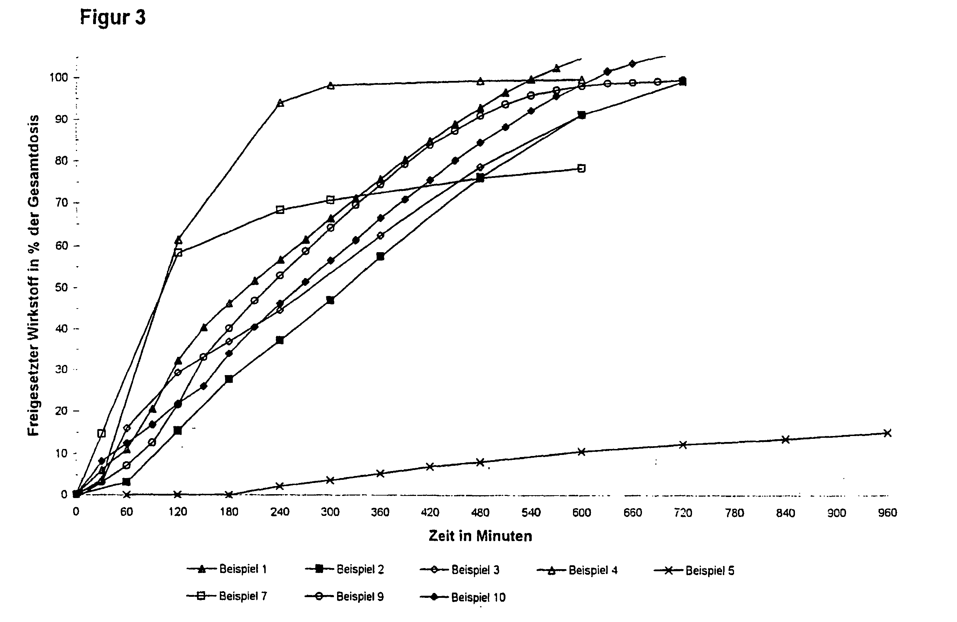

Das Freisetzungsprofil wurde gemäß der oben angegebenen Methode in der Körbchenapparatur bestimmt und ist in der nachfolgenden Tabelle 1 sowie in Figur 3 wiedergegeben.

**Tabelle 1:**

| Zeit (min) | Freigesetztes Tramadolsaccharinat in % der Gesamtdosis an Wirkstoff |
|---|---|
| 0 | 0 |
| 60 | 11 |
| 120 | 32 |
| 180 | 46 |
| 240 | 57 |
| 300 | 67 |
| 360 | 76 |
| 480 | 93 |
| 600 | 105 |

Desweiteren wurde die Wirkstofffreisetzung aus den so hergestellten, mit Retardüberzug versehenen Pellets gemäß der vorstehend angegebenen Methode in Freisetzungsmedien unterschiedlicher Zusammensetzung gemäß der nachstehenden Tabelle 2 geprüft:

**Tabelle 2:**

| | |
|---|---|
| a) | 2 Stunden Magensaft bei pH 1,2 und 10 Stunden Darmsaft bei pH 7,2 |
| b) | 10 Stunden Magensaft bei pH 1,2 |
| c) | 10 Stunden Darmsaft bei pH 6,0 |
| d) | 10 Stunden Darmsaft bei pH 7,2 |
| e) | 2 Stunden Magensaft bei pH 1,2 und 10 Stunden Darmsaft bei pH 7,2 mit jeweils 0,1 % Tween 80® |

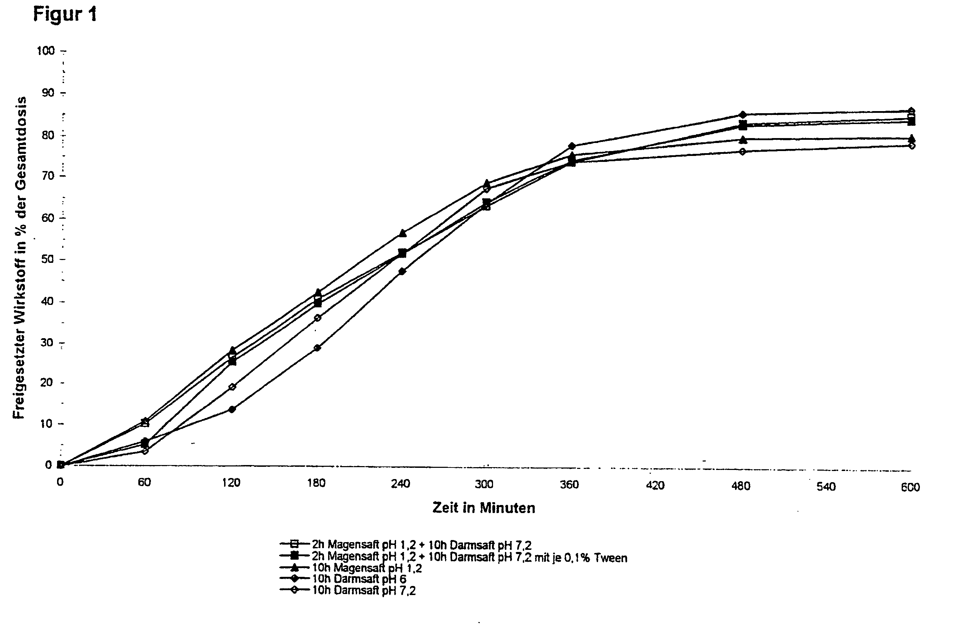

Die Ergebnisse dieser Untersuchungen sind in Figur 1 dargestellt. Wie aus Figur 1 ersichtlich ist, wird das Freisetzungsprofil der mit einem Retardüberzug versehenen Tramadolsaccharinat-Pellets durch die unterschiedliche Zusammensetzung des Freisetzungsmediums kaum beeinflußt. Weder der pH - Wert, noch die Ionenstärke, noch die Anwesenheit von oberflächenaktiven Substanzen beeinflussen das Freisetzungsprofil der mit dem Retardüberzug versehenen Pellets.

### Vergleichsbeispiel 1:

### Herstellung der Pellets enthaltend Tramadolhydrochlorid:

275 g Tramadolhydrochlorid, 75 g mikrokristalline Cellulose (Avicel PH 105®, FMC) und 150 g niedrig substituierte Hydroxypropylcellulose (1-HPC LH31®) werden 10 Minuten in einem Kennwood Chef Planetenmischer gemischt und anschließend mit 250 g demineralisiertem Wasser 10 Minuten granuliert. Das feuchte Granulat wird im NICA E140 Extruder mit einer 1,0 x 2,0 mm Extrusionsmatrize extrudiert und das feuchte Extrudat im NICA Spheronizer 15 Minuten bei 800 UPM spheronisiert. Die Pellets werden anschließend über Nacht bei 50°C im Trockenschrank getrocknet.

Die Ausbeute an Pellets mit einer Partikelgröße im Bereich von 800 bis 1250 µm beträgt über 90%.

### Aufbringen des Überzuges:

320 g dieser Pellets (800 - 1250 µm) wurden in der Wirbelschicht (Aeromatic, Strea 1) mit einer wäßrigen Dispersion der nachstehenden Zusammensetzung bei einer Produkttemperatur von 26 bis 32°C bis zu einer Gewichtszunahme von 25 Gew.-% (bezogen auf das Gewicht der Ausgangspellets) überzogen. Dieser Überzugsauftrag entspricht einem Polymerauftrag von 16 Gew.-% (bezogen auf das Gewicht der Ausgangspellets). Die Pellets werden anschließend 10 Minuten in der Wirbelschicht bei 45°C getrocknet.

| Wäßrige Dispersion für 320 g Pellets: | |
|---|---|
| Eudragit RS 30 D® | 152,8 g |
| Eudragit RL 30 D® | 13,9 g |
| Triethylcitrat | 15,0 g |
| Talkum | 15,0 g |
| Demineralisiertes Wasser | 203,3 g |
| Gesamt: | 400,0 g |

228 mg mit Retardüberzug versehene Pellets enthalten 100 mg Tramadolhydrochlorid.

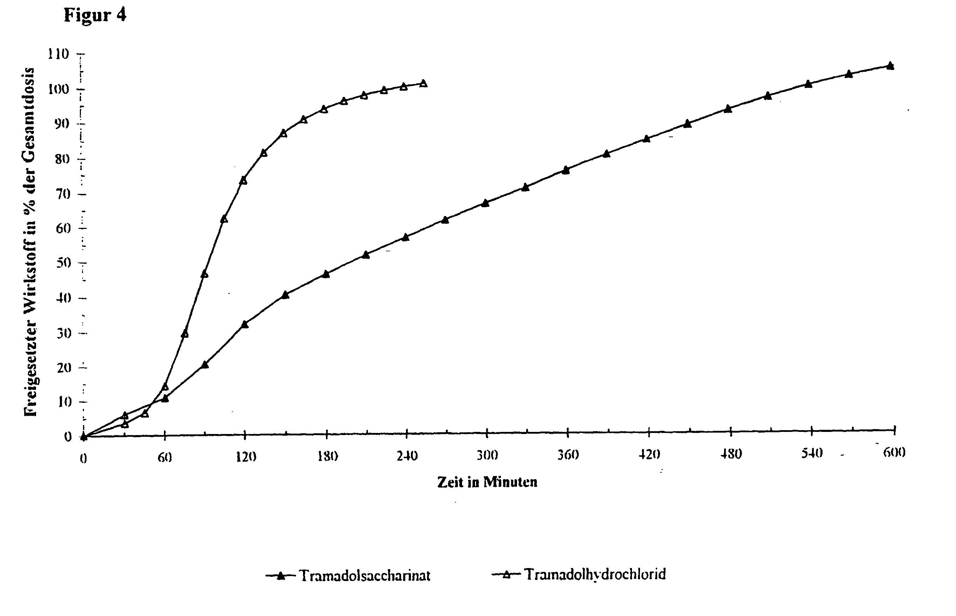

Das Freisetzungsprofil wurde gemäß der oben angegebenen Methode in der Körbchenapparatur bestimmt. Die Ergebnisse dieser Untersuchungen sowie der für die erfindungsgemäßen Pellets gemäß Beispiel 1 sind in Figur 4 wiedergegeben.

Wie aus Figur 4 ersichtlich ist, zeigen die mit einem Retardüberzug versehenen Tramadolsaccharinat-Pellets eine Wirkstofffreisetzung über 10 Stunden, während die mit einem Retardüberzug versehenen Tramadolhydrochlorid-Pellets trotz des dickeren Polymerüberzugs und der weniger permeablen Polymerzusammensetzung des Überzuges nach einer kurzen Verzögerungszeit den Wirkstoff nahezu vollständig bereits innerhalb von 4 Stunden freisetzen.

Weiterhin wurde die intrinsische Wirkstoff-Freisetzung des Wirkstoffes Tramadolhydrochlorid bzw. Tramadolsaccharinat bestimmt.

Die Bestimmung der intrinsischen Wirkstofffreisetzung erfolgt sowohl für Tramadolhydrochlorid als auch für Tramadolsaccharinat in demineralisiertem Wasser (37°C ± 0,5°C) entsprechend der Methode "<1087> intrinsic dissolution" veröffentlicht in USP 24-NF19 first supplement (S. 2706 f). Für jeden der beiden Wirkstoffe wird die intrinsische Freisetzung aus der Steigung der kumulativen Wirkstofffreisetzung bis einschließlich des Zeitpunktes, an dem 10% des Preßlings freigesetzt worden sind, berechnet und als Freisetzungsrate in mg · min⁻¹ · cm⁻² nachstehend aufgeführt.

| Intrinsische Freisetzung in Wasser: | |
|---|---|
| Tramadolhydrochlorid: | 21 mg · min⁻¹ · cm⁻² |
| Tramadolsaccharinat: | 2 mg · min⁻¹ · cm⁻² |

Der Unterschied in der intrinsischen Wirkstoff-Freisetzung zeigt deutlich, daß Tramadolsaccharinat wesentlich langsamer aus Arzzneiformen freigesetzt wird als Tramadolhydrochlorid und deshalb auch langsamer durch Diffusionsbarrieren permeiert.

### Beispiel 2:

### Herstellung der Pellets:

505 g Tramadolsaccharinat und 505 g mikrokristalline Cellulose (Avicel PH 105®, FMC) werden für 10 Minuten im Kennwood Chef Planetenmischer gemischt und anschließend mit 630 g demineralisiertem Wasser 10 Minuten granuliert. Das feuchte Granulat wird im NICA E140 Extruder mit einer 1,0 x 2,0 mm Extrusionsmatrize extrudiert und das feuchte Extrudat im NICA Spheronizer 15 Minuten bei 800 UPM spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

200 g dieser Pellets (800 - 1250 µm) werden in der Wirbelschicht (Aeromatic, Strea 1) mit einer wäßrigen Dispersion der nachstehenden Zusammensetzung bei einer Produkttemperatur von 26 bis 30°C bis zu einer Gewichtszunahme von 27 Gew-% (bezogen auf das Gewicht der Ausgangspellets) überzogen. Dieser Überzug entspricht einem Polymerauftrag von 21 Gew.-% (bezogen auf das Gewicht der Ausgangspellets). Die überzogenen Pellets werden anschließend 10 Minuten in der Wirbelschicht bei 45°C getrocknet.

| Wäßrige Dispersion für 200 g Pellets: | |
|---|---|
| Eudragit RS 30 D® | 108,6 g |
| Eudragit RL 30 D® | 30,6 g |
| Triethylcitrat | 8,3 g |
| Glycerinmonostearat | 2,1 g |
| Tween 80® | 0,2 g |
| Demineralisiertes Wasser | 111,2 g |
| Gesamt: | 261,0 g |

379 mg mit Retardüberzug versehene Pellets enthalten 149 mg Tramadolsaccharinat, entsprechend einer Wirkstoffmenge von 100 mg Tramadolhydrochlorid.

Das Freisetzungsprofil wurde gemäß der obenstehend beschriebenen Methode in der Körbchenapparatur bestimmt und ist in der nachfolgenden Tabelle 3 sowie in Figur 3 wiedergegeben. Abweichend von den obenstehend beschriebenen Bedinungen wurden die überzogenen Pellets 10 Stunden in künstlichem Darmsaft bei pH 7,2 geprüft.

**Tabelle 3:**

| Zeit (min) | Freigesetztes Tramadolsaccharinat in % der Gesamtdosis an Wirkstoff |
|---|---|
| 0 | 0 |
| 60 | 3 |
| 120 | 14 |
| 180 | 26 |
| 240 | 35 |
| 300 | 46 |
| 360 | 56 |
| 480 | 74 |
| 600 | 91 |
| 720 | 100 |

### Beispiel 3:

### Herstellung der Pellets:

505 g Tramadolsaccharinat und 505 g mikrokristalline Cellulose (Avicel PH 105®, FMC) werden für 10 Minuten im Kennwood Chef Planetenmischer gemischt und anschließend mit 630 g demineralisiertem Wasser 10 Minuten granuliert. Das feuchte Granulat wird im NICA E140 Extruder mit einer 1,0 x 2,0 mm Extrusionsmatrize extrudiert und das feuchte Extrudat im NICA Spheronizer 15 Minuten bei 800 UPM spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

200 g dieser Pellets (800 - 1250 µm) werden in der Wirbelschicht (Aeromatic, Strea 1) mit einer wäßrigen Dispersion der nachstehenden Zusammensetzung bei einer Produkttemperatur von 26 bis 30°C bis zu einer Gewichtszunahme von 21 Gew-% (bezogen auf das Gewicht der Ausgangspellets) überzogen. Dieser Überzugsauftrag entspricht einem Polymerauftrag von 14 Gew.-% (bezogen auf das Gewicht der Ausgangspellets). Die überzogenen Pellets werden entweder direkt nach dem Überziehen und dem üblichen Trocknen bzw. nach einer Temperung bei 40°C über 15 Stunden im Trockenschrank der Freisetzungsprüfung unterzogen.

| Wäßrige Dispersion für 200 g Pellets: | |
|---|---|
| Eudragit RS 30 D® | 80,0 g |
| Eudragit RL 30 D® | 20,0 g |
| Triethylcitrat | 6,0 g |
| Talkum | 6,0 g |
| Demineralisiertes Wasser | 88,0 g |
| Gesamt: | 200,0 g |

361 mit Retardüberzug versehene Pellets enthalten 149 mg Tramadolsaccharinat, entsprechend einer Wirkstoffmenge von 100 mg Tramadolhydrochlorid.

Die jeweiligen Freisetzungsprofile wurden gemäß der obenstehend beschriebenen Methode in der Körbchenapparatur bestimmt und sind in der nachfolgenden Tabelle 4 sowie für die ungetemperten Pellets in Figur 3 wiedergegeben.

**Tabelle 4:**

| Zeit (min) | Freigesetztes Tramadolsaccharinat in % der Gesamtdosis an Wirkstoff **ohne Tempern** | Freigesetztes Tramadolsaccharinat in % der Gesamtdosis an Wirkstoff **15 h Temperung bei 40°C** |
|---|---|---|
| 0 | 0 | 0 |
| 60 | 16 | 15 |
| 120 | 29 | 27 |
| 180 | 37 | 34 |
| 240 | 45 | 41 |
| 360 | 63 | 57 |
| 480 | 79 | 73 |
| 600 | 92 | 87 |

Die Unterschiede zwischen den Werten für die Wirkstoff-Freisetzung ohne und mit Temperung von circa 5% entsprichen der üblichen Streuung der Meßwerte. Die Temperung nach dem Aufbringen des Überzuges ist daher ohne Einfluß auf das Freisetzungsprofil.

### Beispiel 4:

### Herstellung der Pellets:

252, 5 g Tramadolsaccharinat und 252,5 g mikrokristalline Cellulose (Avicel PH 105®, FMC) werden für 10 Minuten in einem Kennwood Chef Planetenmischer gemischt und anschließend mit 338,0 g demineralisiertem Wasser für 10 Minuten granuliert. Das feuchte Granulat wird im NICA E140 Extruder mit einer 1,0 x 2,0 mm Extrusionsmatrize extrudiert und das feuchte Extrudat im NICA Spheronizer 15 Minuten bei 800 UPM spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

170 g dieser Pellets (800 - 1250 µm) werden in der Wirbelschicht (Aeromatic, Strea 1) mit einer wäßrigen Dispersion der nachfolgenden Zusammensetzung bei einer Produkttemperatur von 26 bis 30°C bis zu einer Gewichtszunahme von 28% Gew.-% (bezogen auf das Gewicht der Ausgangspellets) überzogen. Dieser Überzugsauftrag entspricht einem Polymerauftrag von 20% Gew.-% (bezogen auf das Gewicht der Ausgangspellets). Die überzogenen Pellets werden anschließend 10 Minuten in der Wirbelschicht bei 45°C getrocknet.

| Wäßrige Dispersion für 170 g Pellets: | |
|---|---|
| Eudragit RS 30 D® | 68,1 g |
| Eudragit RL 30 D® | 45,4 g |
| Triethylcitrat | 6,8 g |
| Talkum | 6,8 g |
| Demineralisiertes Wasser | 99,9 g |
| Gesamt: | 227,0 g |

381 mg mit Retardüberzug versehene Pellets enthalten 149 mg Tramadolsaccharinat, entsprechend einer Wirkstoffmenge von 100 mg Tramadolhydrochlorid.

Das Freisetzungsprofil wurde gemäß der oben angegebenen Methode in der Körbchenapparatur bestimmt und ist in der nachfolgenden Tabelle 5 sowie in Figur 3 wiedergegeben. Abweichend von den obenstehend beschriebenen Bedingungen wurden die überzogenen Pellets 6 Stunden in künstlichem Darmsaft bei pH 7,2 geprüft.

**Tabelle 5:**

| Zeit (min) | Freigesetztes Tramadolsaccharinat in % der Gesamtdosis an Wirkstoff |
|---|---|
| 0 | 0 |
| 30 | 4 |
| 120 | 62 |
| 240 | 94 |
| 300 | 99 |
| 480 | 100 |

### Beispiel 5:

### Herstellung der Pellets:

505,0 g Tramadolsaccharinat und 505,0 g mikrokristalline Cellulose (Avicel PH 105®, FMC) werden 10 Minuten lang in einem Kennwood Chef Planetenmischer gemischt und anschließend mit 630,0 g demineralisiertem Wasser 10 Minuten granuliert. Das feuchte Granulat wird im NICA E140 Extruder mit einer 1,0 x 2,0 mm Extrusionsmatrize extrudiert und das feuchte Extrudat im NICA Spheronizer 15 Minuten bei 800 UPM spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

200 g dieser Pellets (800 - 1250 µm) werden in der Wirbelschicht (Aeromatic, Strea 1) mit einer wäßrigen Dispersion der nachfolgenden Zusammensetzung bei einer Produkttemperatur von 26 bis 30°C bis zu einer Gewichtszunahme von 20% Gew.-% (bezogen auf das Gewicht der Ausgangspellets) überzogen. Dieser Überzugsauftrag entspricht einem Polymerauftrag von 16% Gew.-% (bezogen auf das Gewicht der Ausgangspellets). Die überzogenen Pellets werden anschließend 10 Minuten in der Wirbelschicht bei 45°C getrocknet.

| Wäßrige Dispersion für 200 g Pellets: | |
|---|---|
| Eudragit RS 30 D® | 106,1 g |
| Triethylcitrat | 6,4 g |
| Glycerinmonostearat | 1,6 g |
| Tween 80® | 0,2 g |
| Demineralisiertes Wasser | 84,7 g |
| Gesamt: | 199,0 g |

358 mg mit Retardüberzug versehene Pellets enthalten 149 mg Tramadolsaccharinat, entsprechend einer Wirkstoffmenge von 100 mg Tramadolhydrochlorid.

Das Freisetzungsprofil wurde nach der obenstehend angegebenen Methode in der Körbchenapparatur bestimmt und ist in der nachfolgenden Tabelle 6 sowie in Figur 3 wiedergegeben. Abweichend von den obenstehend beschriebenen Bedinungen wurden die überzogenen Pellets 14 Stunden in künstlichem Darmsaft bei pH 7,2 geprüft.

**Tabelle 6:**

| Zeit (min) | Freigesetztes Tramadolsaccharinat in % der Gesamtdosis an Wirkstoff |
|---|---|
| 0 | 0 |
| 60 | 0 |
| 120 | 0 |
| 180 | 0 |
| 240 | 2 |
| 300 | 3 |
| 360 | 5 |
| 420 | 7 |
| 480 | 8 |
| 600 | 10 |
| 720 | 12 |
| 840 | 14 |
| 960 | 15 |

### Beispiel 6:

### Untersuchung der Lagerstabilität von mit einem Retardüberzug versehenen Tramadolsaccharinat-Pellets:

Die gemäß Beispiel 1 hergestellten mit einem Retardüberzug versehenen Pellets wurden jeweils in geschlossenen Gläsern für 1 Woche bzw. für 2 Wochen entweder bei 30 °C und 70 % relativer Luftfeuchtigkeit, bei 40 °C trocken oder bei 40°C und 75% relativer Luftfeuchtigkeit gelagert und jeweils das Freisetzungsprofil gemäß der obenstehend beschriebenen Methode nach der Lagerung bestimmt. Abweichend von den obenstehend beschriebenen Bedinungen wurden die überzogenen Pellets 10 Stunden in künstlichem Darmsaft bei pH 7,2 geprüft.

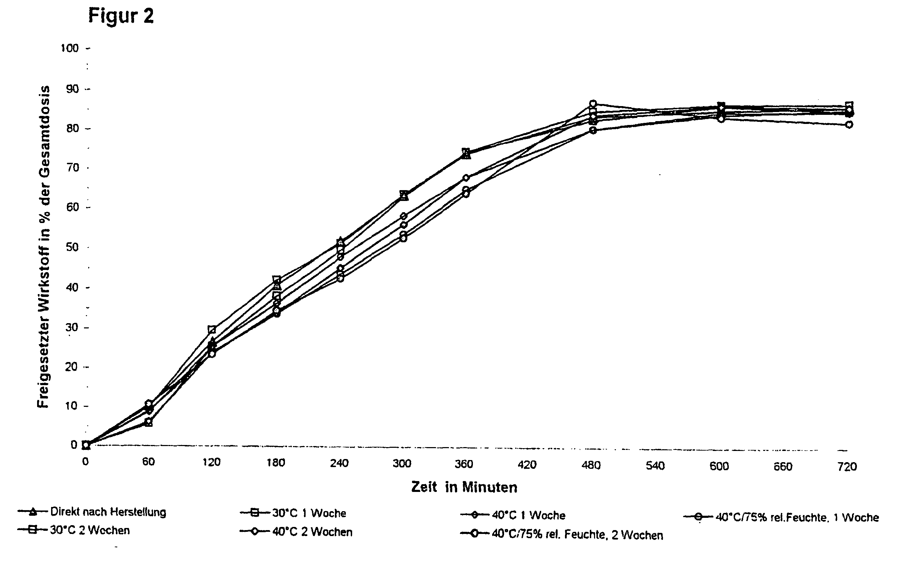

Die Ergebnisse dieser jeweiligen Untersuchungen sind in Figur 2 dargestellt. Wie aus Figur 2 ersichtlich ist, zeigt die Lagerung bei 30°C und 70 % relativer Luftfeuchtigkeit sowie bei 40°C trocken keinerlei Einfluß auf das Wirkstoff-Freisetzungsprofil. Auch nach Lagerung bei 40 °C und 75 % relativer Luftfeuchtigkeit kommt es lediglich zu einer geringfügigen Verlangsamung der Freisetzung aus den zum Teil verklebten Pellets. Die erfindungsgemäßen Darreichungsformen des Tramadolsaccharinates zeigen daher unmittelbar nach ihrer Herstellung ein retardiertes Freisetzungsprofil des Wirkstoffes, das ohne Tempern lagerstabil ist.

### Beispiel 7:

### Herstellung der Pellets:

250 g Tramadolsaccharinat und 250 g mikrokristalline Cellulose (Avicel PH 105®) werden 10 Minuten in einem Kennwood Chef Planetenmischer gemischt und anschließend mit der 443 g demineralisiertem Wasser 10 Minuten granuliert. Das feuchte Granulat wird im NICA E140 Extruder mit einer 1,0 x 2,0 mm Extrusionsmatrize extrudiert und das feuchte Extrudat anschließend im NICA Spheronizer 15 Minuten bei 800 UPM spheronisiert. Die Pellets werden anschließend über Nacht bei 50°C im Trockenschrank getrocknet. Die Ausbeute an Pellets mit einer Partikelgröße im Bereich von 800 bis 1250 µm beträgt über 90%.

### Aufbringen des Überzuges:

170 g dieser Pellets (800 - 1250 µm) werden in der Wirbelschicht (Aeromatic, Strea 1) mit einer wäßrigen Dispersion der nachstehenden Zusammensetzung bei einer Produkttemperatur von 35 bis 40°C bis zu einer Gewichtszunahme von 5 Gew.-% (bezogen auf das Gewicht der Ausgangspellets) überzogen. Die überzogenen Pellets werden anschließend zwei Stunden bei 60°C im Trockenschrank getrocknet.

| Wäßrige Dispersion für 170 g Pellets: | |
|---|---|
| Surelease E-7-7050®* | 34,0 g |
| Dem. Wasser | 23,0 g |
| Gesamt: | 57,0 g |

| | |
|---|---|
| * 25%ige wäßrige Ethylcellulosepseudolatexdispersion. Die handelsübliche Fertigdispersion enthält Dibutylsebacat als Weichmacher und Siliciumdioxid als Gleitmittel. | |

313 mg mit Retardüberzug versehene Pellets enthalten 149 mg Tramadolsaccharinat, entsprechend einer Dosierung von 100 mg Tramadolhydrochlorid.

Das Freisetzungsprofil wurde nach der oben angegebenen Methode in der Körbchenapparatur bestimmt und ist in der nachfolgenden Tabelle 7 sowie in Figur 3 wiedergegeben.

**Tabelle 7:**

| Zeit (min) | Freigesetztes Tramadolsaccharinat in % der Gesamtdosis an Wirkstoff |
|---|---|
| 0 | 0 |
| 30 | 15 |
| 120 | 58 |
| 240 | 69 |
| 300 | 71 |
| 480 | 76 |
| 600 | 79 |

### Beispiel 8:

### Herstellung von Tabletten:

| Zusammensetzung pro Tablette: | |
|---|---|
| Tramadolsaccharinat | 149 mg |
| Cellactose | 146 mg |
| Magnesiumstearat | 3 mg |

Die Cellactose und das Magnesiumstearat werden gesiebt und anschließend mit dem Tramadolsaccharinat 10 Minuten in einem Freifallmischer (Bohle, LM 40) homogen gemischt. Dieses Gemisch wird auf einer Korsch EKO Exzenterpresse mit einem Stempelwerkzeug zu 10 mm großen Draggee-gewölbten Tabletten mit einer Höhe von' ca. 5 mm und einem Wölbungsradius von 8 mm verpreßt.

### Aufbringen des Überzuges:

2000 dieser Tabletten werden in der Wirbelschicht (Aeromatic, Strea 1) mit einer wäßrigen Dispersion der nachstehenden Zusammensetzung bei einer Produkttemperatur von 26 bis 30°C bis zu einer Gewichtszunahme von 6,5 Gew.-% (bezogen auf das Gewicht der Ausgangstablette) überzogen. Dieser Überzugsauftrag entspricht einem Polymerauftrag von 5,0 Gew.-% (bezogen auf das Gewicht der Ausgangstablette). Die überzogenen Tabletten werden 10 Minuten bei 40°C im Trockenschrank getrocknet.

| Wäßrige Dispersion für 2000 Tabletten: | |
|---|---|
| Eudragit RS 30 D® | 69,7 g |
| Eudragit RL 30 D® | 29,8 g |
| Triethylcitrat | 6,0 g |
| Glycerinmonostearat | 1,5 g |
| Tween 80® | 0,1 g |
| Demineralisiertes Wasser | 92,4 g |
| Gesamt: | 199,5 g |

Das Freisetzungsprofil wurde nach der obenstehend angegebenen Methode in der Blattrührapparatur bestimmt und ist in der untenstehenden Tabelle 8 wiedergegeben.

### Vergleichsbeispiel 2:

### Herstellung von Tabletten:

| Zusammensetzung pro Tablette: | |
|---|---|
| Tramadolhydrochlorid | 100 mg |
| Cellactose | 98 mg |
| Magnesiumstearat | 2 mg |

Die Cellactose und das Magnesiumstearat werden gesiebt und anschließend mit dem Tramadolsaccharinat 10 Minuten in einem Freifallmischer (Bohle, LM 40) homogen gemischt. Dieses Gemisch wird auf einer Korsch EKO Exzenterpresse mit einem Stempelwerkzeug zu 9 mm großen Draggee-gewölbten Tabletten mit einer Höhe von ca. 5 mm und einem Wölbungsradius von 8 mm verpreßt.

### Aufbringen des Überzuges:

2000 dieser Tabletten werden in der Wirbelschicht (Aeromatic, Strea 1) mit einer wäßrigen Dispersion der nachstehenden Zusammensetzung bei einer Produkttemperatur von 26 bis 30°C bis zu einer Gewichtszunahme von 6,5 Gew.-% (bezogen auf das Gewicht der Ausgangstablptte) überzogen. Dieser Überzugsauftrag entspricht einem Polymerauftrag von 5,0 Gew.-% (bezogen auf das Gewicht der Ausgangstablette). Die überzogenen Tabletten werden 10 Minuten bei 40°C im Trockenschrank getrocknet.

| Wäßrige Dispersion für 2985 Tabletten: | |
|---|---|
| Eudragit RS 30 D® | 69,7 g |
| Eudragit RL 30 D® | 29,8 g |
| Triethylcitrat | 6,0 g |
| Glycerinmonostearat | 1,5 g |
| Tween 80® | 0,1 g |
| Demineralisiertes Wasser | 92,4 g |
| Gesamt: | 199,5 g |

Das Freisetzungsprofil wurde nach der obenstehend angegebenen Methode in der Blattrührapparatur bestimmt und ist in der nachstehenden Tabelle 8 wiedergegeben.

**Tabelle 8:**

| Zeit (min) | Freigesetztes Tramadolsaccharinat in % der Gesamtdosis an Wirkstoff | Freigesetztes Tramadolhydrochlorid in % der Gesamtdosis an Wirkstoff |
|---|---|---|
| 0 | 0 | 0 |
| 30 | 1 | 2 |
| 240 | 9 | 91 |
| 480 | 17 | 96 |
| 720 | 25 | 98 |

Während der Wirkstoff Tramadolhydrochlorid aus den überzogenen Tabletten innerhalb von 4 Stunden bereits zu > 90 % freigesetzt wird, erfolgt die Freisetzung des Tramadalsaccharinates aus den überzogenen Tabletten wesentlich langsamer und mit konstanter Freisetzungsrate, wobei nach 12 Stunden erst 25 % der Gesamtdosis freigesetzt werden.
Auch hierbei zeigt sich deutlich, daß die langsamere Lösungsgeschwindigkeit des Tramadolsaccharinates bei identischen Diffusionsbarrieren zu einer stärkeren Retardierung führt als bei Tramadolhydrochlorid und im Gegensatz zu diesem bereits bei niedrigeren Filmschichtdicken zu einer Freisetzungskinetik 0. Ordnung führt.

### Beispiel 9:

### Herstellung der Pellets:

500 g Tramadolhydrochlorid, 345 g Natriumsaccharinat und 845 g mikrokristalline Cellulose (Avicel PH 101®) werden 10 Minuten gemischt und anschließend mit einer zur Granulation ausreichenden Menge demineralisiertem Wasser 10 Minuten granuliert. Nach der Granulation wird das Granulat mit einem Nica E 140 Extruder mit einer 0,8 x 1,6 mm Extrusionsmatrize extrudiert und das Extrudat erneut mit einer ausreichenden Menge an Wasser granuliert, so daß eine plastische Masse entsteht. Diese wird nochmals extrudiert. Das feuchte Extrudat wird im Nica Spheroniser Typ S450 spheronisiert. Nach der Trocknung der Pellets im Trockenschrank werden diese klassiert, wobei > 90% der Pellets eine Größe im Bereich von 0,63 bis 1,0 mm aufweisen.

### Aufbringen der Überzüge:

1000 g dieser Pellets werden in der Wirbelschicht (Hüttlin HKC05) bei einer Produkttemperatur von 26 bis 30°C bis zu einer Gewichtszunahme von 20 Gew.-% (bezogen auf das Gewicht der Ausgangspellets) mit einer wäßrigen Dispersion der nachstehenden Zusammensetzung überzogen. Dieser Überzugsauftrag entspricht einem Polymerauftrag von 16 Gew.-% (bezogen auf das Gewicht der Ausgangspellets). Die Pellets werden anschließend 10 Minuten in der Wirbelschicht bei 45°C getrocknet.

| Wäßrige Dispersion für 1000 g Pellets: | |
|---|---|
| Eudragit RS30D® | 499,5 g |
| Eudragit RL30D® | 166,5 g |
| Triethylcitrat | 40,0 g |
| Glycerinmonostearat | 10,0 g |
| Tween 80® | 1,1 g |
| Demineralisiertes Wasser | 612,9 g |
| Gesamt: | 1330 g |

### Abfüllung der überzogenen Pellets in Kapseln:

406 mg der mit einem Retardüberzug versehenen Pellets (entsprechend einem Wirkstoffgehalt von 100 mg Tramadolhydrochlorid) werden auf einer Zanasi E6 Steckkapselmaschine in Hartgelatinekapseln der Größe 0 gefüllt.

Das Freisetzungsprofil wurde gemäß der oben angegebenen Methode in der Körbchenapparatur bestimmt und ist in der nachfolgenden Tabelle 9 und in Figur 3 wiedergegeben. Abweichend von den obenstehend beschriebenen Bedinungen wurden die überzogenen Pellets 10 Stunden in künstlichem Darmsaft bei pH 7,2 geprüft.

**Tabelle 9:**

| Zeit in min | Freigesetztes Tramadolsaccharinat in % der Gesamtdosis an Wirkstoff |
|---|---|
| 0 | 0 |
| 60 | 7 |
| 120 | 22 |
| 240 | 53 |
| 480 | 91 |
| 600 | 99 |
| 720 | 100 |

### Beispiel 10:

Die Herstellung der Pellets erfolgt gemäß Beispiel 4.

400 g dieser Pellets (800 - 1250 µm) werden in der Wirbelschicht (Aeromatic. Strea 1) mit einer wäßrigen Dispersion der nachstehenden Zusammensetzung bei einer Produkttemperatur von 26 bis 30 °C bis zu einer Gewichtszunahme von 25 Gew.-% (bezogen auf das Gewicht der Ausgangspellets) überzogen. Dieser Überzugsauftrag entspricht einem Polymerauftrag von 20 Gew.-% (bezogen auf das Gewicht der Ausgangspellets). Die überzogenen Pellets werden 10 Minuten bei 40°C in der Wirbelschichtapparatur getrocknet.

| Wäßrige Dispersion für 400 g Pellets: | |
|---|---|
| Eudragit RS 30 D® | 200,0 g |
| Eudragit RL 30 D® | 66,7 g |
| Triethylcitrat | 16,0 g |
| Glycerinmonostearat | 4,0 g |
| Tween 80® | 0,5 g |
| Demineralisiertes Wasser | 212,8 g |
| Gesamt: | 500,0 g |

373 mg mit einem Retardüberzug versehene Pellets enthalten 149 mg Tramadolsaccharinat, entsprechend einer Dosierung von 100 mg Tramadolhydrochlorid.

### Herstellung von Tabletten:

| Zusammensetzung pro Tablette: | |
|---|---|
| überzogene Pellets | 373 mg |
| Kollidon CL® | 22,4 mg |
| Avicel PH101® | 142,0 mg |
| Magnesiumstearat | 2,6 mg |
| Gesamt: | 540 mg |

Die mit einem Retardüberzug versehehen Pellets werden 5 Minuten in einem Freifallmischer (Bohle, LM 40) mit Kollidon CL® (quervernetztes Polyvinylpyrrolidon) gemischt und anschließend weitere 10 Minuten mit mikrokristalliner Cellulose (Avicel PH 101®) und Magnesiumstearat vermischt. Diese Mischung wird auf einer Tablettenrundläuferpresse (Fette, P1200) zu runden, biplanen Tabletten mit einem Durchmesser von 12 mm, einem Gewicht von jeweils 500 mg und einer Härte von 100 bis 130 N verpreßt.

In wäßrigem Medium zerfallen diese Tabletten innerhalb von 1-2 Minuten in die einzelnen Pellets.

Das Freisetzungsprofil wurde gemäß der obenstehend beschriebenen Methode in der Blattrührerapparatur bestimmt und ist in der nachfolgenden Tabelle 10 sowie in Figur 3 wiedergegeben.

**Tabelle 10:**

| Zeit (min) | Freigesetztes Tramadolsaccharinat in % der Gesamtdosis an Wirkstoff |
|---|---|
| 0 | 0 |
| 30 | 8 |
| 120 | 22 |
| 240 | 46 |
| 300 | 57 |
| 480 | 86 |
| 600 | 102 |

### Beispiel 11:

Die Herstellung der Pellets erfolgt gemäß Beispiel 2.

200 g dieser Pellets (800 - 1250 µm) werden in der Wirbelschicht (Aeromatic, Strea 1) mit einer wäßrigen Dispersion der nachstehenden Zusammensetzung bei einer Produkttemperatur von 35 bis 40°C bis zu einer Gewichtszunahme von 20 Gew.-% (bezogen auf das Gewicht der Ausgangspellets) überzogen. Dieser Überzugsauftrag entspricht einem Polymerauftrag von 15 Gew.-% (bezogen auf das Gewicht der Ausgangspellets). Die überzogenen Pellets werden 10 Minuten bei 45°C im Wirbelschichtgerät getrocknet.

| Wäßrige Dispersion für 200 g Pellets: | |
|---|---|
| Kollicoat® RS 30 D | 100,0 g |
| Propylenglykol | 3,0 g |
| Talkum | 7,0 g |
| Demineralisiertes Wasser | 90,0 g |
| Gesamt: | 200,0 g |

358 mg mit einem Retardüberzug versehene Pellets enthalten 149 mg Tramadolsaccharinat, entsprechend einer Dosierung von 100 mg Tramadolhydrochlorid.

Das Freisetzungsprofil wurde gemäß der oben angegebenen Methode in der Körbchenapparatur bestimmt und ist in der nachfolgenden Tabelle 11 wiedergegeben.

**Tabelle 11:**

| Zeit (min) | Freigesetztes Tramadolsaccharinat in % der Gesamtdosis an Wirkstoff |
|---|---|
| 0 | 0 |
| 30 | 6 |
| 120 | 37 |
| 180 | 54 |
| 240 | 61 |
| 300 | 71 |
| 480 | 76 |
| 600 | 92 |
| 720 | 98 |

Figur 3 zeigt eine Auswahl unterschiedlicher Freisetzungsprofile für den Wirkstoff Tramadolsaccharinat aus Extrusionspellets, die aus wäßriger Dispersion überzogen wurden. In Abhängigkeit von der Filmzusammensetzung und der Filmschichtdicke lassen sich geeignete Wirkstoff-Freisetzungsprofile für eine Formulierung zur einmaligen Applikation pro Tag bzw. zur zweimaligen Applikation pro Tag erzielen, sowie die Freisetzungskinetik von einer Kinetik 1. Ordnung zu einer Kinetik 0. Ordnung verschieben. Dies zeigt die große Vielfalt an erzielbaren Wirkstoff-Freisetzungsprofilen wobei nur handelsübliche Polymerdispersionen in der vom Hersteller empfohlenen Verarbeitung ohne weitere Maßnahmen oder besondere Zusatzstoffe eingesetzt werden.

## Patentansprüche

1. Mit wenigstens einem retardierenden Überzug versehene Tramadol-Darreichungsformen enthaltend den Wirkstoff Tramadol als Tramadolsaccharinat und ggf. weitere Hilfsstoffe.

2. Darreichungsformen gemäß Anspruch 1, **dadurch gekennzeichnet, daß** die Tramadol-Verbindung als eine in situ gebildete Verbindung vorliegt.

3. Darreichungsform gemäß Anspruch 1 oder 2, **dadurch gekennzeichnet, daß** zur Herstellung der Tramadol-Verbindung ein wasserlösliches, pharmazeutisch verträgliches Salz des Tramadols, vorzugsweise Tramadolhydrochlorid. und ein wasserlösliches, pharmazeutisch verträgliches Salz des Saccharins, vorzugsweise das Natrium-, Kalium-, Kalzium- oder Ammoniumsalz. besonders bevorzugt das Natriumsalz eingesetzt worden ist.

4. Darreichungsformen gemäß einem der Ansprüche 1 bis 3 zur oralen Applikation.

5. Darreichungsformen gemäß Anspruch 4, **dadurch gekennzeichnet, daß** sie in Form von Tabletten, Kapseln oder Suspensionen vorliegen

6. Darreichungsformen gemäß Anspruch 4, **dadurch gekennzeichnet, daß** sie in multipartikulärer Form, vorzugsweise in Form von Mikrotabletten, Mikrokapseln, Mikropellets, Granulaten, Wirkstoffkristallen oder Pellets, ggf. in Kapseln abgefüllt oder zu Tabletten verpreßt, oder in hydrophiler oder lipophiler Flüssigkeit vorliegen.

7. Darreichungsformen gemäß Anspruch 6, **dadurch gekennzeichnet, daß** die Granulate oder Pellets eine Größe im Bereich von 0,1 bis 3 mm, vorzugsweise im Bereich von 0,5 bis 2 mm aufweisen.

8. Darreichungsformen gemäß Anspruch 6, **dadurch gekennzeichnet, daß** die Mikrotabletten einen Durchmesser von 0,5 bis 5 mm, vorzugsweise 1 bis 3 mm, besonders bevorzugt 1 bis 2 mm aufweisen.

9. Darreichungsformen gemäß Anspruch 6, **dadurch gekennzeichnet, daß** die Wirkstoffkristalle, Mikropartikel, Mikropellets oder Mikrokapseln einen Durchmesser von 10 µm bis 1 mm, vorzugsweise 15 µm bis 0,5 mm, besonders bevorzugt 30 µm bis 200 µm aufweisen.

10. Darreichungsformen gemäß einem der Ansprüche 1 bis 9, **dadurch gekennzeichnet, daß** der retardierende Überzug auf einem wasserunlöslichen ggf. modifizierten natürlichen oder synthetischen Polymeren oder auf einem natürlichen, halbsynthetischen oder synthetischen Wachs oder Fett oder Fettalkohol oder einem Gemisch aus wenigstens zwei dieser Komponenten basiert.

11. Darreichungsformen gemäß Anspruch 10, **dadurch gekennzeichnet, daß** als wasserunlösliche Polymere Poly(meth)acrylate, bevorzugt Poly(C₁₋₄)-Alkyl(meth)acrylate, Poly(C₁₋₄)-dialkylamino-(C₁₋₄)-alkyl(meth)acrylate und/oder deren Copolymere, bevorzugt Ethylacrylat/Methylmethacrylat-Copolymere mit einem molaren Verhältnis der Monomeren von 2 : 1 (Eudragit NE30D®), Ethylacrylat/Methylmethacrylat/Trimethylammoniumethylmethacrylat-chlorid-Copolymere mit einem molaren Verhältnis der Monomeren von 1 : 2 : 0,1 (Eudragit RS®), Ethylacrylat/Methylmethacrylat/Trimethylammoniumethylmethacrylatchlorid-Copolymere mit einem molaren Verhältnis der Monomeren von 1 : 2 : 0,2 (Eudragit RL®) oder ein Gemisch aus wenigstens zwei dieser vorstehend genannten Polymeren als Überzugsmaterial vorliegen.

12. Darreichungsform gemäß Anspruch 10, **dadurch gekennzeichnet, daß** als wasserunlösliche Polymere Cellulosederivate, vorzugsweise Alkylcellulose, besonders bevorzugt Ethylcellulose, oder Celluloseester, vorzugsweise Celluloseacetat als Überzugsmaterial vorliegen.

13. Darreichungsformen gemäß Anspruch 11 oder 12, **dadurch gekennzeichnet, daß** die Polymere aus wäßrigem Medium, vorzugsweise aus wäßriger Latex oder Pseudolatex-Dispersionen aufgetragen worden sind.

14. Darreichungsformen gemäß Anspruch 10, **dadurch gekennzeichnet, daß** als Überzugs-Polymere ein Gemisch aus Polyvinylacetat und Polyvinylpyrollidon, vorzugsweise in Form wäßriger Pseudolatex-Dispersionen eingesetzt worden ist.

15. Darreichungsformen gemäß Anspruch 10, **dadurch gekennzeichnet, daß** als Wachs Carnaubawachs, Bienenwachs, Glycerinmonostearat, Glycerinmonobehenat (Compritol ATO888®), Glycerinditripalmitostearat (Precirol ATO5®), mikrokristallines Wachs oder ein Gemisch aus wenigstens zwei dieser Komponenten, vorzugsweise durch Schmelzüberzugsverfahren als Überzugsmaterial aufgetragen worden ist.

16. Darreichungsformen gemäß einem der Ansprüche 11 bis 13, **dadurch gekennzeichnet, daß** die Polymere in Kombination mit üblichen Weichmachern eingesetzt worden sind.

17. Darreichungsformen gemäß Anspruch 16, **dadurch gekennzeichnet, daß** als Weichmacher lipophile Diester aus aliphatischen oder aromatischen Dicarbonsäuren mit C₆-C₄₀ und aliphatischen Alkoholen mit C₁-C₈, vorzugsweise Dibutylphthalat, Diethylphthalat, Dibutylsebacat oder Diethylsebacat, ein hydrophile oder lipophile Ester der Zitronensäure, vorzugsweise Triethylcitrat, Tributylcitrat, Acetyltributylcitrat oder Acetyltriethylcitrat, Polyethylenglycole, Propylenglycole, Ester des Glycerins, vorzugsweise Triacetin, acetylierte Monound Diglyceride, mittelkettige Triglyceride, Ölsäure oder ein Gemisch aus wenigstens zwei dieser Weichmacher eingesetzt worden sind.

18. Darreichungsformen gemäß Anpruch 16 oder 17, **dadurch gekennzeichnet, daß** der Weichmacher in Mengen von 5 bis 50 Gew-%., vorzugsweise 10 bis 40 Gew.-%, besonders bevorzugt 10 bis 30 Gew-%, bezogen auf das polymere Überzugsmaterial, vorliegt.

19. Darreichungsformen gemäß einem der Ansprüche 1 bis 18, **dadurch gekennzeichnet, daß** sie eine retardierende Matrix aufweisen.

20. Darreichungsformen gemäß Anspruch 19, **dadurch gekennzeichnet, daß** die Matrix auf einem hydrophilen Matrixmaterial, vorzugsweise hydrophilen Polymeren, besonders bevorzugt auf Celluloseethern, Celluloseestern und/oder Acrylharzen, ganz besonders bevorzugt auf Ethylcellulose, Hydroxypropylmethylcellulose, Hydroxypropylcellulose, Hydroxymethylcellulose, Poly(meth)acrylsäure und/oder deren Salzen, Amiden und/oder Estern basiert.

21. Darreichungsform gemäß Anspruch 19 oder 20, **dadurch gekennzeichnet, daß** die Matrix auf einem hydrophoben Matrixmaterial, vorzugsweise hydrophoben Polymeren, Wachsen, Fetten, langkettigen Fettsäuren, Fettalkoholen oder entsprechenden Estern oder Ethern oder deren Gemischen, besonders bevorzugt auf Mono- oder Diglyceriden von C₁₂-C₃₀-Fettsäuren und/oder C₁₂-C₃₀-Fettalkoholen und/oder Wachsen oder deren Gemischen basiert.

22. Darreichungsformen gemäß einem der Ansprüche 1 bis 21, **dadurch gekennzeichnet, daß** sie als weiteren Überzug einen magensaftresistenten Überzug aufweisen.

23. Darreichungsformen gemäß Anspruch 22, **dadurch gekennzeichnet, daß** der magensaftresistente Überzug aus Methacrylsäure/Methylmethacrylat-Copolymeren mit einem molaren Verhältnis der Monomeren von 1 : 1 (Eudragit L®), Methacrylsäure/Methylmethacrylat-Copolymeren mit einem molaren Verhältnis der Monomeren von 1 : 2 (Eudragit S®), Methacrylsäure/Ethylacrylat-Copolymeren mit einem molaren Verhältnis der Monomeren von 1 : 1 (Eudragit L30-D55®) Methacrylsäure/Methylacrylat/Methylmethacrylat mit einem molaren Verhältnis der Monomeren von 7 : 3 : 1 (Eudragit FS®), Schellack, Hydroxypropylmethylcelluloseacetatsuccinat, Celluloseacetatphthalat oder aus einer Mischung aus wenigstens zwei dieser Komponenten, ggf. auch in Kombination mit Poly(meth)acrylaten, vorzugsweise Eudragit NE30D® und/oder Eudragit RL® und/oder Eudragit RS® besteht.

24. Darreichungsformen gemäß einem der Ansprüche 1 bis 23, **dadurch gekennzeichnet, daß** sie den Wirkstoff Tramadol zusätzlich in unretardierter Form enthalten.

25. Darreichungsformen gemäß einem der Ansprüche 1 bis 23, **dadurch gekennzeichnet, daß** sie ein retardiertes, lagerstabiles Freisetzungsprofil für den Wirkstoff Tramadol unmittelbar nach deren Herstellung, ohne Temperung aufweisen.

26. Darreichungsformen gemäß einem der Ansprüche 1 bis 25, **dadurch gekennzeichnet, daß** der retardierende Überzug aus wäßrigen Medien aufgebracht und nach der üblichen Trocknung nicht getempert worden ist.

27. Darreichungsformen gemäß einem der Ansprüche 1 bis 26 für eine einmalige Applikation pro Tag, **dadurch gekennzeichnet, daß** sie 10 bis 1200 mg, , vorzugsweise 20 bis 1000 mg, besonders bevorzugt 100 bis 800 mg Tramadol enthalten.

28. Darreichungsformen gemäß einem der Ansprüche 1 bis 26 für eine zweimalige Applikation pro Tag, **dadurch gekennzeichnet, daß** sie 5 bis 600 mg, vorzugsweise 10 bis 500 mg, besonders bevorzugt 50 bis 400 mg Tramadol enthalten.

29. Darreichungsformen gemäß einem der Ansprüche 1 bis 28 zur Bekämpfung von Schmerzen.

30. Darreichungsformen gemäß einem der Ansprüche 1 bis 28 zur Behandlung von Harninkontinenz.

31. Darreichungsformen gemäß einem der Ansprüche 1 bis 28 zur Behandlung von Husten.

32. Darreichungsformen gemäß einem der Ansprüche 1 bis 28 zur Behandlung von inflammatorischen und/oder allergischen Reaktionen.

33. Darreichungsformen gemäß einem der Ansprüche 1 bis 28 zur Behandlung von Depressionen.

34. Darreichungsformen gemäß einem der Ansprüche 1 bis 28 zur Behandlung von Drogen- und/oder Alkoholmißbrauch.

35. Darreichungsformen gemäß einem der Ansprüche 1 bis 28 zur Behandlung von Gastritis.

36. Darreichungsformen gemäß einem der Ansprüche 1 bis 28 zur Behandlung von Diarrhoe.

37. Darreichungsformen gemäß einem der Ansprüche 1 bis 28 zur Behandlung von cardiosvaskulären Erkrankungen.

38. Darreichungsformen gemäß einem der Ansprüche 1 bis 28 zur Behandlung von Atemwegserkrankungen.

39. Darreichungsformen gemäß einem der Ansprüche 1 bis 28 zur Behandlung von seelischen Erkrankungen.

40. Darreichungsformen gemäß einem der Ansprüche 1 bis 28 zur Behandlung von Epilepsie.

## Claims

1. Forms of administration of tramadol, provided with at least one sustained-release coating, which contain the active substance tramadol as tramadol saccharinate, optionally together with other auxiliary substances.

2. Forms of administration according to Claim 1, **characterized in that** the tramadol compound is present as a compound formed in situ.

3. Form of administration according to Claim 1 or 2, **characterized in that** the tramadol compound has been prepared using a water-soluble, pharmaceutically acceptable salt of tramadol, preferably tramadol hydrochloride, and a water-soluble, pharmaceutically acceptable salt of saccharin, preferably the sodium, potassium, calcium or ammonium salt and particularly preferably the sodium salt.

4. Forms of administration according to one of Claims 1 to 3 for oral administration.

5. Forms of administration according to Claim 4, **characterized in that** they are tablets, capsules or suspensions.

6. Forms of administration according to Claim 4, **characterized in that** they are multiparticulate, preferably in the form of microtablets, microcapsules, micropellets, granules, active substance crystals or pellets, optionally filled into capsules or compressed to tablets, or in a hydrophilic or lipophilic liquid.

7. Forms of administration according to Claim 6, **characterized in that** the granules or pellets have a size in the range 0.1 to 3 mm, preferably in the range 0.5 to 2 mm.

8. Forms of administration according to Claim 6, **characterized in that** the microtablets have a diameter of 0.5 to 5 mm, preferably 1 to 3 mm and particularly preferably 1 to 2 mm.

9. Forms of administration according to Claim 6, **characterized in that** the active substance crystals, microparticles, micropellets or microcapsules have a diameter of 10 µm to 1 mm, preferably 15 µm to 0.5 mm and particularly preferably 30 µm to 200 µm.

10. Forms of administration according to one of Claims 1 to 9, **characterized in that** the sustained-release coating is based on a water-insoluble, optionally modified, natural or synthetic polymer, on a natural, semisynthetic or synthetic wax or fat or fatty alcohol, or on a mixture of at least two of these components.

11. Forms of administration according to Claim 10, **characterized in that** the water-insoluble polymers present as the coating material are poly (meth) acrylates, preferably poly (C₁₋₄) alkyl (meth) acrylates, poly (C₁₋₄) dialkylamino (C₁₋₄) alkyl (meth)acrylates and/or copolymers thereof, preferably ethyl acrylate/methyl methacrylate copolymers with a monomer molar ratio of 2:1 (Eudragit NE30D®), ethyl acrylate/methyl methacrylate/trimethylammonium ethyl methacrylate-chloride copolymers with a monomer molar ratio of 1:2:0.1 (Eudragit RS®), ethyl acrylate/methyl methacrylate/trimethylammonium ethyl methacrylate-chloride copolymers with a monomer molar ratio of 1:2:0.2 (Eudragit RL®), or a mixture of at least two of the above-mentioned polymers.

12. Form of administration according to Claim 10, **characterized in that** the water-insoluble polymers present as the coating material are cellulose derivatives, preferably alkyl cellulose and particularly preferably ethyl cellulose, or cellulose esters, preferably cellulose acetate.

13. Forms of administration according to Claim 11 or 12, **characterized in that** the polymers have been applied from an aqueous medium, preferably from an aqueous latex or pseudolatex dispersions.

14. Forms of administration according to Claim 10, **characterized in that** the coating polymer which has been used is a mixture of polyvinyl acetate and polyvinylpyrrolidone, preferably in the form of aqueous pseudolatex dispersions.

15. Forms of administration according to Claim 10, **characterized in that** the wax which has been applied as the coating material, preferably by the hot-melt coating process, is carnauba wax, beeswax, glycerol monostearate, glycerol monobehenate (Compritol ATO888®), glycerol ditripalmitostearate (Precirol ATO5®), microcrystalline wax or a mixture of at least two of these components.

16. Forms of administration according to one of Claims 11 to 13, **characterized in that** the polymers have been used in combination with conventional plasticizers.

17. Forms of administration according to Claim 16, **characterized in that** the plasticizers which have been used are lipophilic diesters of C₆-C₄₀ aliphatic or aromatic dicarboxylic acids and C₁₋C₈ aliphatic alcohols, preferably dibutyl phthalate, diethyl phthalate, dibutyl sebacate or diethyl sebacate, a hydrophilic or lipophilic citric acid ester, preferably triethyl citrate, tributyl citrate, acetyltributyl citrate or acetyltriethyl citrate, polyethylene glycols, propylene glycols, glycerol esters, preferably triacetin, acetylated mono- and diglycerides, medium-chain triglycerides, oleic acid or a mixture of at least two of these plasticizers.

18. Forms of administration according to Claim 16 or 17, **characterized in that** the plasticizer is present in amounts of 5 to 50 wt.%, preferably 10 to 40 wt.% and particularly preferably 10 to 30 wt.%, based on the polymeric coating material.

19. Forms of administration according to one of Claims 1 to 18, **characterized in that** they have a sustained-release matrix.

20. Forms of administration according to Claim 19, **characterized in that** the matrix is based on a hydrophilic matrix material, preferably hydrophilic polymers, particularly preferably cellulose ethers, cellulose esters and/or acrylic resins and very particularly preferably ethyl cellulose, hydroxypropyl methyl cellulose, hydroxypropyl cellulose, hydroxymethyl cellulose, poly(meth)acrylic acid and/or salts, amides and/or esters thereof.

21. Form of administration according to Claim 19 or 20, **characterized in that** the matrix is based on a hydrophobic matrix material, preferably hydrophobic polymers, waxes, fats, long-chain fatty acids, fatty alcohols or corresponding esters or ethers, or mixtures thereof, and particularly preferably C₁₂-C₃₀ fatty acid mono- or diglycerides and/or C₁₂-C₃₀ fatty alcohols and/or waxes, or mixtures thereof.

22. Forms of administration according to one of Claims 1 to 21, **characterized in that** they have an enteric coating as an additional coating.

23. Forms of administration according to Claim 22, **characterized in that** the enteric coating consists of methacrylic acid/methyl methacrylate copolymers with a monomer molar ratio of 1:1 (Eudragit L®), methacrylic acid/methyl methacrylate copolymers with a monomer molar ratio of 1:2 (Eudragit S®), methacrylic acid/ethyl acrylate copolymers with a monomer molar ratio of 1:1 (Eudragit L30-D55®), methacrylic acid/methyl acrylate/methyl methacrylate with a monomer molar ratio of 7:3:1 (Eudragit FS®), shellac, hydroxypropyl methyl cellulose acetate-succinate, cellulose acetate-phthalate or a mixture of at least two of these components, optionally also in combination with poly(meth)acrylates, preferably Eudragit NE30D® and/or Eudragit RL® and/or Eudragit RS®.

24. Forms of administration according to one of Claims 1 to 23, **characterized in that** they additionally contain the active substance tramadol in non-retarded form.

25. Forms of administration according to one of Claims 1 to 23, **characterized in that** their retarded release profile for the active substance tramadol immediately after preparation is stable on storage without tempering.

26. Forms of administration according to one of Claims 1 to 25, **characterized in that** the sustained-release coating has been applied from aqueous media and not tempered after the conventional drying.

27. Forms of administration according to one of Claims 1 to 26 to be taken once a day, **characterized in that** they contain 10 to 1200 mg, preferably 20 to 1000 mg and particularly preferably 100 to 800 mg of tramadol.

28. Forms of administration according to one of Claims 1 to 26 to be taken twice a day, **characterized in that** they contain 5 to 600 mg, preferably 10 to 500 mg and particularly preferably 50 to 400 mg of tramadol.

29. Forms of administration according to one of Claims 1 to 28 for the control of pain.

30. Forms of administration according to one of Claims 1 to 28 for the treatment of urinary incontinence.

31. Forms of administration according to one of Claims 1 to 28 for the treatment of coughs.

32. Forms of administration according to one of Claims 1 to 28 for the treatment of inflammatory and/or allergic reactions.

33. Forms of administration according to one of Claims 1 to 28 for the treatment of depression.

34. Forms of administration according to one of Claims 1 to 28 for the treatment of drug and/or alcohol abuse.

35. Forms of administration according to one of Claims 1 to 28 for the treatment of gastritis.

36. Forms of administration according to one of Claims 1 to 28 for the treatment of diarrhoea.

37. Forms of administration according to one of Claims 1 to 28 for the treatment of cardiovascular disease.

38. Forms of administration according to one of Claims 1 to 28 for the treatment of respiratory disease.

39. Forms of administration according to one of Claims 1 to 28 for the treatment of mental illness.

40. Forms of administration according to one of Claims 1 to 28 for the treatment of epilepsy.

## Revendications

1. Formes d'administration de tramadol pourvues d'au moins un revêtement retardateur, contenant la substance active tramadol sous forme de saccharinate de tramadol et, le cas échéant, d'autres substances auxiliaires.

2. Formes d'administration suivant la revendication 1, **caractérisées en ce que** le composé tramadol est présent comme composé formé *in situ*.

3. Formes d'administration suivant la revendication 1 ou 2, **caractérisées en ce qu'**un sel hydrosoluble acceptable du point de vue pharmaceutique du tramadol, avantageusement le chlorhydrate de tramadol, et un sel hydrosoluble acceptable du point de vue pharmaceutique de la saccharine, avantageusement le sel de sodium, de potassium, de calcium ou d'ammonium, notamment le sel de sodium, ont été utilisés pour la préparation du composé de tramadol.

4. Formes d'administration suivant l'une des revendications 1 à 3, destinées à l'administration orale.

5. Formes d'administration suivant la revendication 4, **caractérisées en ce qu'**elles se présentent sous forme de comprimés, de gélules ou de suspensions.

6. Formes d'administration suivant la revendication 4, **caractérisées en ce qu'**elles se présentent sous une forme multiparticulaire, avantageusement sous forme de microcomprimés, de microcapsules, de micropellets, de granulés, de cristaux de substance active ou de pellets, éventuellement chargés dans des gélules ou pressés en comprimés, ou sous forme de liquide hydrophile ou lipophile.

7. Formes d'administration suivant la revendication 6, **caractérisées en ce que** les granulés ou pellets ont des dimensions comprises dans une plage de 0,1 à 3 mm, avantageusement dans la plage de 0,5 à 2 mm.

8. Formes d'administration suivant la revendication 6, **caractérisées en ce que** les microcomprimés ont un diamètre de 0,5 à 5 mm, avantageusement de 1 à 3 mm, notamment de 1 à 2 mm.

9. Formes d'administration suivant la revendication 6, **caractérisées en ce que** les cristaux de substance active, les microparticules, les micropellets ou les microgélules ont un diamètre de 10 µm à 1 mm, avantageusement de 15 µm à 0,5 mm, notamment de 30 µm à 200 µm.

10. Formes d'administration suivant l'une des revendications 1 à 9, **caractérisées en ce que** le revêtement retardateur est à base d'un polymère naturel ou synthétique insoluble dans l'eau, éventuellement modifié, ou à base d'une cire ou d'une graisse ou d'un alcool gras naturel, semi-synthétique ou synthétique ou d'un mélange d'au moins deux de ces composants.

11. Formes d'administration suivant la revendication 10, **caractérisées en ce qu'**elles comprennent comme polymères insolubles dans l'eau constituant la matière de revêtement, des poly(méth)acrylates, avantageusement des poly(alkyle en C₁ à C₄) (méth) acrylates, des polydi (alkyle en C₁ à C₄) amino-(alkyle en C₁ à C₄) (méth)acrylates, et/ou leurs copolymères, avantageusement des copolymères acrylate d'éthyle/méthacrylate de méthyle ayant un rapport molaire des monomères de 2:1 (Eudragit NE30D®), des copolymères acrylate d'éthyle/méthacrylate de méthyle/chlorure de méthacrylate de triméthylammoniumméthyle ayant un rapport molaire des monomères de 2:2:0,1 (Eudragit RS®), des copolymères acrylate d'éthyle/méthacrylate de méthyle/chlorure de méthacrylate de triméthylammoniumméthyle ayant un rapport molaire des monomères de 1:2:0,2 (Eudragit RL®) ou un mélange d'au moins deux des polymères mentionnés ci-dessus.

12. Forme d'administration suivant la revendication 10, **caractérisée en ce qu'**elle comprend comme polymères insolubles dans l'eau des dérivés de cellulose, avantageusement une alkylcellulose, notamment l'éthylcellulose, ou des esters de cellulose, avantageusement l'acétate de cellulose, comme matière de revêtement.

13. Formes d'administration suivant la revendication 11 ou 12, **caractérisées en ce que** les polymères ont été appliqués à partir d'un milieu aqueux, avantageusement à partir d'un latex aqueux ou de dispersions d'un pseudolatex.

14. Formes d'administration suivant la revendication 10, **caractérisées en ce qu'**un mélange de poly(acétate de vinyle) et de polyvinylpyrrolidone a été utilisé comme polymère de revêtement, avantageusement sous forme de dispersions aqueuses d'un pseudolatex.

15. Formes d'administration suivant la revendication 10, **caractérisées en ce que** la cire qui a été appliquée comme matière de revêtement est la cire de carnauba, la cire d'abeille, le monostéarate de glycérol, le monobéhénate de glycérol (Compritol ATO888®), le ditripalmitostéarate de glycérol (Precirol ATO5®), une cire microcristalline ou un mélange d'au moins deux de ces composants, avantageusement par des procédés de revêtement à l'état fondu.

16. Formes d'administration suivant l'une des revendications 11 à 13, **caractérisées en ce que** les polymères ont été utilisés en association avec des plastifiants usuels.

17. Formes d'administration suivant la revendication 16, **caractérisées en ce que** les plastifiants qui ont été utilisés sont des diesters lipophiles formés par des diacides carboxyliques aliphatiques ou aromatiques en C₆ à C₄₀ avec des alcools aliphatiques en C₆ à C₈, avantageusement le phtalate de dibutyle, le phtalate de diéthyle, de sébacate de dibutyle ou le sébacate de diéthyle, un ester hydrophile ou lipophile d'acide citrique, avantageusement le citrate de triéthyle, le citrate de tributyle et le citrate d'acétyltributyle ou le citrate d'acétyltriéthyle, des polyéthylèneglycols, des propylèneglycols, des esters de glycérol, avantageusement la triacétine, des monoglycérides et diglycérides acétylés, des triglycérides de moyenne longueur de chaîne, l'acide oléique ou un mélange d'au moins deux de ces plastifiants.

18. Formes d'administration suivant la revendication 16 ou 17, **caractérisées en ce que** le plastifiant est présent en quantités de 5 à 50% en poids, avantageusement de 10 à 40% en poids, notamment de 10 à 30% en poids par rapport à la matière polymère de revêtement.

19. Formes d'administration suivant l'une des revendications 1 à 18, **caractérisées en ce qu'**elles présentent une matrice retardatrice.

20. Formes d'administration suivant la revendication 19, **caractérisées en ce que** la matrice est à base d'une matière hydrophile, avantageusement de polymères hydrophiles, très avantageusement d'éthers de cellulose, d'esters de cellulose et/ou de résines acryliques, notamment à base d'éthylcellulose, d'hydroxypropylméthylcellulose, d'hydroxypropylcellulose, d'hydroxyméthylcellulose, de polymère d'acide(méth)acrylique et/ou de ses sels, amides et/ou esters.

21. Forme d'administration suivant la revendication 19 ou 20, **caractérisée en ce que** la matrice est à base d'une matière hydrophobe, avantageusement de polymères, cires, matières grasses, acides gras à longue chaîne, alcools gras ou esters ou éthers hydrophobes correspondants ou de leurs mélanges, en particulier à base de monoglycérides ou de diglycérides, d'acides gras en C₁₂ à C₃₀ et/ou d'alcools gras en C₁₂ à C₃₀ et/ou de cires, ou leurs mélanges.

22. Formes d'administration suivant l'une des revendications 1 à 21, **caractérisées en ce qu'**elles présentent comme autre revêtement un revêtement résistant au suc gastrique.

23. Formes d'administration suivant la revendication 22, **caractérisées en ce que** le revêtement résistant au suc gastrique est constitué de copolymères acide méthacrylique/méthacrylate de méthyle ayant un rapport molaire des monomères de 1:1 (Eudragit L®), de copolymères acide méthacrylique/méthacrylate de méthyle ayant un rapport molaire des monomères de 1:2 ((Eudragit S®), de copolymères acide méthacrylique/acrylate d'éthyle ayant un rapport molaire des monomères de 1:1 (Eudragit L30-D55®), d'acide méthacrylique/acrylate de méthyle/méthacrylate de méthyle ayant un rapport molaire des monomères de 7:3:1 (Eudragit FS®), de gomme-laque, d'acétate-succinate d'hydroxypropylméthylcellulose, d'acétate-phtalate de cellulose, ou d'un mélange d'au moins deux de ces composants, le cas échéant également en association avec des poly(méth)acrylates, avantageusement l'Eudragit NE30D® et/ou l'Eudragit RL® et/ou l'Eudragit RS®.

24. Formes d'administration suivant l'une des revendications 1 à 23, **caractérisées en ce qu'**elles contiennent en outre la substance active tramadol sous forme non retardée.

25. Formes d'administration suivant l'une des revendications 1 à 23, **caractérisées en ce qu'**elles présentent un profil de libération retardée stable au stockage pour la substance active tramadol immédiatement après sa préparation, sans durcissement à la chaleur.

26. Formes d'administration suivant l'une des revendications 1 à 25, **caractérisées en ce que** le revêtement retardateur est appliqué à partir de milieux aqueux et n'est pas durci à la chaleur après le séchage usuel.

27. Formes d'administration suivant l'une des revendications 1 à 26, conçues pour une administration une fois par jour, **caractérisées en ce qu'**elles contiennent 10 à 1200 mg, avantageusement 20 à 1000 mg, notamment 100 à 800 mg de tramadol.

28. Formes d'administration suivant l'une des revendications 1 à 26, conçues pour une administration deux fois par jour, **caractérisées en ce qu'**elles contiennent 5 à 600 mg, avantageusement 10 à 500 mg, notamment 50 à 400 mg de tramadol.

29. Formes d'administration suivant l'une des revendications 1 à 28, destinées à combattre des douleurs.

30. Formes d'administration suivant l'une des revendications 1 à 28, destinées au traitement de l'incontinence urinaire.

31. Formes d'administration suivant l'une des revendications 1 à 28, destinées au traitement de la toux.

32. Formes d'administration suivant l'une des revendications 1 à 28, destinées au traitement de réactions inflammatoires et/ou allergiques.

33. Formes d'administration suivant l'une des revendications 1 à 28, destinées au traitement de dépressions.

34. Formes d'administration suivant l'une des revendications 1 à 28, destinées au traitement de l'abus de drogue et/ou d'alcool.

35. Formes d'administration suivant l'une des revendications 1 à 28, destinées au traitement de la gastrite.

36. Formes d'administration suivant l'une des revendications 1 à 28, destinées au traitement de la diarrhée.

37. Formes d'administration suivant l'une des revendications 1 à 28, destinées au traitement de maladies cardiovasculaires.

38. Formes d'administration suivant l'une des revendications 1 à 28, destinées au traitement de maladies des voies respiratoires.

39. Formes d'administration suivant l'une des revendications 1 à 28, destinées au traitement de maladies psychiques.

40. Formes d'administration suivant l'une des revendications 1 à 28, destinées au traitement de l'épilepsie.
